# EUROPEAN PATENT SPECIFICATION

(11) **EP 3 678 659 B1**
(45) Date of publication and mention of the grant of the patent: **17.12.2025**
(21) Application number: 18779490.4
(22) Date of filing: 05.09.2018
(51) Int. Cl.: A61K 31/4178, A61K 31/444, A61K 31/46, A61K 31/5517, A61K 45/06, A61P 25/08, A61P 21/02, A61P 39/02

(54) **METHODS OF USING DANTROLENE TO TREAT EXPOSURE TO A NEUROTOXIC AGENT**
VERWENDUNG VON DANTROLEN ZUR BEHANDLUNG EINER NERVENGIFTEXPOSITION
MÉTHODES D'UTILISATION DU DANTROLÈNE POUR TRAITER UNE EXPOSITION À UN AGENT NEUROTOXIQUE

(30) Priority: 05.09.2017 US 201762554049 P; 21.05.2018 US 201862674406 P
(43) Date of publication of application: 15.07.2020
(73) Proprietor: Eagle Pharmaceuticals, Inc., Woodcliff Lake, NJ 07677 (US)
(72) Inventor: HEPNER, Adrian, Woodcliff Lake, NJ 07677 (US)
(74) Representative: Carpmaels & Ransford LLP
(86) International application number: PCT/US2018/049515
(87) International publication number: WO 2019/050923

(56) References cited:
- G.P.H BALLOUGH ET AL: "A Viable Neuroprotection Strategv Following- Soman-induced Status Epilepticus", 1 December 2003 (2003-12-01), XP055524775, Retrieved from the Internet <URL:http://www.dtic.mil/dtic/tr/fulltext/u2/a443565.pdf> [retrieved on 20181119]
- NEWMARK JONATHAN ET AL: "Dantrolene plus diazepam: A viable strategy for neuroprotection following soman-induced status epilepticus", NEUROLOGY, LIPPINCOTT WILLIAMS & WILKINS, PHILADELPHIA, US, vol. 60, no. 5 Supplement 1, 11 March 2003 (2003-03-11), pages A385, XP009509427, ISSN: 0028-3878

## Description

### TECHNICAL FIELD

The disclosure is directed to methods of treating subjects exposed to neurotoxic nerve agents with dantrolene, or a pharmaceutically acceptable salt thereof.

### BACKGROUND

Neurotoxic nerve agents, also referred to as nerve agents, are organophosphate compounds that disrupt the transmission of nerve impulses in the nervous system by blocking acetylcholinesterase. These agents, whether in gaseous, aerosol, or liquid form, are extremely toxic and have a very rapid effect. Nerve agent poisoning produces a myriad of symptoms including unilateral or bilateral miosis, headache, convulsions, loss of consciousness, coma, and death. In particular, nerve agent poisoning produces seizures, which can quickly develop into status epilepticus (SE), which leads to seizure-related brain damage. Nerve agent-induced seizures usually become refractory to standard antiepileptic therapy.

Medical care for treating nerve agent poisoning will likely be delayed beyond the therapeutic window of opportunity to terminate nerve agent-induced seizures, and seizure-related brain damage will continue along the pathological cascade. Thus, there is a need for drug therapy that is capable of interrupting the pathologic cascade to provide neuroprotection during the refractory phase of nerve agent-induced seizures.
G.P.H Ballough at al.: "A Viable Neuroprotection Strategy Following Soman- induced Status Epilepticus", 1 December 2003 discloses the neuroprotective effectiveness of dantrolene in the prevention of soman-induced SRBD.
Newmark Jonathan et al.: "Dantrolene plus diazepam: A viable strategy for neuroprotection following soman-induced status epilepticus", Neurology, vol. 60, no. 5 Supplement 1, 11 March 2003 discloses that dantrolene/mannitol appears to act synergistically with diazepam in this animal model.

### SUMMARY

The disclosure is directed to a pharmaceutical composition comprising an amount of dantrolene or a pharmaceutically acceptable salt thereof for use in methods of treating subjects exposed to a neurotoxic nerve agent as defined in the appended claims.

### BRIEF DESCRIPTION OF THE DRAWINGS

Figure 1 depicts the mean body weight of test group over time.
Figure 2A depicts Sucrose Preference Test results from Study Day 14.
Figure 2B depicts Sucrose Preference Test results from Study Day 15.
Figure 3 depicts results from the Forced Swim Test.
Figure 4 depicts mean necrosis scores from the fronto-parietal cortex.
Figure 5A depicts photomicrograph from the fronto-parietal cortex of an animal in Group E (positive control, water 50 minutes post SE onset). Arrows mark faded, shrunken neurons. Necrosis score is 4.
Figure 5B depicts photomicrograph from the fronto-parietal cortex of an animal in Group D (30 mg/kg RYANODEX^{®}, 20 minutes post SE onset). Arrows mark normal neurons. Necrosis score is 0.

### DETAILED DESCRIPTION OF ILLUSTRATIVE EMBODIMENTS

The invention is defined by the appended claims. Any subject-matter falling outside the scope of the claims is provided for information purposes, only. The references to methods of treatment in this description are to be interpreted as references to the compounds, pharmaceutical compositions and medicaments of the present invention for use in a method for treatment of the human (or animal) body by therapy.

The present disclosure may be understood more readily by reference to the following detailed description taken in connection with the accompanying figures and examples, which form a part of this disclosure. Also, as used in the specification, including the appended claims, the singular forms "a," "an," and "the" include the plural, and reference to a particular numerical value includes at least that particular value, unless the context clearly dictates otherwise. All ranges are inclusive and combinable.

The modifier "about" should be considered as disclosing the range defined by the absolute values of the two endpoints. For example, the expression "from about 2 to about 4" also discloses the range "from 2 to 4." When used to modify a single number, the term "about" may refer to plus or minus 10% of the indicated number and includes the indicated number. For example, "about 10%" may indicate a range of 9% to 11%, and "about 1" means from 0.9 to 1.1.

Further, reference to values stated in ranges includes each and every value within that range.

As used herein, the term "pharmaceutical composition" shall mean a composition that is suitable for administration to humans and contains pharmaceutically acceptable excipients, *e.g.,* without limitation, stabilizers, bulking agents, buffers, carriers, diluents, vehicles, solubilizers, and binders. As used herein pharmaceutical composition includes, but is not limited to, a liquid form ready for subcutaneous injection or infusion and intramuscular injection.

"Therapeutically effective amount" refers to an amount of an active pharmaceutical agent described herein which is sufficient to inhibit, halt, or cause an improvement in a disorder or condition being treated in a particular subject or subject population. In certain embodiments, in a human or other mammal, a therapeutically effective amount can be determined experimentally in a laboratory or clinical setting, or may be the amount required by government guidelines for the particular disease and subject being treated.

As used herein, "ameliorate" refers to the lessening of the severity in a disorder or condition being treated in a particular subject or subject population.

As used herein, "patient" or "subject" is intended to mean a mammal. Thus, the methods described herein are applicable to human and nonhuman subjects. In the methods for use of the invention, the subject is a human.

The term "pharmaceutically acceptable" as used herein means that the thing that is pharmaceutically acceptable, e.g., components, including containers, of a pharmaceutical composition, does not cause unacceptable loss of pharmacological activity or unacceptable adverse side effects. Examples of pharmaceutically acceptable components are provided in The United States Pharmacopeia (USP), The National Formulary (NF), adopted at the United States Pharmacopeial Convention, held in Rockville, Md. in 1990 and FDA Inactive Ingredient Guide 1990, 1996 issued by the U.S. Food and Drug Administration. Other grades of solutions or components that meet necessary limits and/or specifications that are outside of the USP/NF may also be used.

The term "pharmaceutically acceptable salt" as used herein means a salt of a compound of the disclosure that is pharmaceutically acceptable and that possesses the desired pharmacological activity of the parent compound. In particular, such salts are non-toxic, and may be inorganic or organic acid addition salts and base addition salts. Specifically, such salts include: salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like.

As used herein, "neurotoxic nerve agent" or "nerve agent" refers to compounds that affect the transmission of nerve impulses in the nervous system. Nerve agents are organophosphorus compounds, that is, they are of the formula (R)₃P(O), wherein each R group can be the same or different. "G"-type nerve agents include O-pinacolyl methylphosphonofluoridate (soman, GD), ethyl N,N-dimethylphosphoramidocyanidate (tabun, GA), propan-2-yl methylphosphonofluoridate (sarin, GB), cyclohexyl methylphosphonofluoridate (cyclosarin, GF), and 2-(Dimethylamino)ethyl (GV). "V"-type nerve agents include O-cyclopentyl S-(2-diethylaminoethyl) methylphosphonothiolate (EA-3148), (S)-(ethyl {[2-(diethylamino)ethyl]sulfonyls}(ethyl)phosphonates) such as (S)-(ethyl {[2-(diethylamino)ethyl]sulfanyl}(ethyl)phosphinate) (VE), O,O-Diethyl S-[2-(diethylamino)ethyl] phosphorothioate (VG), S-[2-(Diethylamino)ethyl] O-ethyl methylphosphonothioate (VM), N,N-diethyl-2-(methyl-(2-methylpropoxy)phosphoryl)sulfanylethanamine (VR), and Ethyl ({2-[bis(propan-2-yl)amino]ethyl}sulfanyl)(methyl)phosphinate (VX). The methods described herein can be used to treat a subject exposed to one nerve agent. The methods described herein can also be used to treat a subject exposed to two or more nerve agents.

As used herein, the phrases, "resulting from exposure to a nerve agent" and "due to nerve agent exposure" refer to effects that are a direct consequence of nerve agent exposure, as well as to effects that are a secondary consequence of nerve agent exposure, as well as to effects that are an indirect consequence of nerve agent exposure.

Among other things, the disclosure is directed to a pharmaceutical composition comprising an amount of dantrolene, or a pharmaceutically acceptable salt thereof, for use in methods of treating a subject exposed to a nerve agent as defined in the appended claims. The invention does not relate to methods of treatment by therapy practice on the human or animal body and any references to such methods are to compositions for use in such methods. For example, in some aspects, the described methods prevent neurologic damage secondary to nerve agent exposure. In other aspects, the described methods provide neuroprotective effects following nerve agent exposure. In other aspects, the described methods ameliorate brain tissue damage secondary to nerve agent exposure. In other aspects, the described methods ameliorate brain tissue damage secondary to status epilepticus secondary to nerve agent exposure. In other aspects, the described methods prevent neuronal necrosis due to nerve agent exposure. In other aspects, the described methods ameliorate neuronal necrosis due to nerve agent exposure. In other aspects, the described methods treat intracellular calcium overload due to nerve agent exposure. In other aspects, the described methods ameliorate intracellular calcium overload due to nerve agent exposure. In other aspects, the described methods prevent intracellular calcium overload due to nerve agent exposure.

'The subjects described herein can be exposed to a nerve agent *via* inhalation. In other aspects, subjects are exposed to a nerve agent *via* transdermal transmission of the agent. In still other aspects, subjects are exposed to a nerve agent *via* consumption of a liquid or food that has been contaminated with a nerve agent. In other aspects, subjects are exposed to a nerve agent *via* subcutaneous, intravenous, or intramuscular administration of the agent to the subject.

In some aspects, the methods are directed to methods of protecting a subject from neural necrosis, after the subject has been exposed to a nerve agent, as described in the appended claims. In these embodiments, a pharmaceutical composition comprising an amount of dantrolene or a pharmaceutically acceptable salt thereof, is administered to the subject after the subject has been exposed to a nerve agent. As used herein, "protection" from neural necrosis encompasses lessening the severity of the effects of the nerve agent or ameliorating the effect of the nerve agent or decreasing the neural damage resulting from the nerve agent exposure. In some aspects, "protection" from neural necrosis encompasses the prevention of neural necrosis in a subject that has been exposed to a nerve agent. That is, subjects that are "protected" from neural necrosis by administration of the described dantrolene-containing compositions perform better on neurobehavioral tests, as compared to nerve agent-exposed subjects that were not administered the described compositions.

In the methods, the fronto-parietal cortex is protected from neural necrosis.

The presence and extent of neural necrosis can be determined using methods known in the art, including neurobehavioral tests, radiological tests, and pathology evaluation.

The disclosure is also directed to methods of protecting a subject from a decrease in central nervous system function resulting from exposure to a nerve agent, as described in the appended claims. These methods comprise administering to the subject a pharmaceutical composition comprising an amount of dantrolene or a pharmaceutically acceptable salt thereof, after the subject has been exposed to a nerve agent.

The disclosure is also directed to methods of protecting a subject from a central nervous system dysfunction resulting from exposure to a nerve agent, as described in the appended claims. These methods comprise administering to the subject a pharmaceutical composition comprising an amount of dantrolene or a pharmaceutically acceptable salt thereof, after the subject has been exposed to a nerve agent.

The disclosure is also directed to methods of treating behavior changes in a subject resulting from exposure to a nerve agent, as described in the appended claims. These methods comprise administering to the subject a pharmaceutical composition comprising an amount of dantrolene or a pharmaceutically acceptable salt thereof, after the subject has been exposed to a nerve agent.

As used herein, "protection" from a decrease in central nervous system function encompasses lessening the severity of the central nervous system effects of the nerve agent or ameliorating the central nervous system effects of the nerve agent or decreasing the central nervous system effects of the nerve agent. That is, subjects that are "protected" from a decrease in central nervous system function by administration of the described compositions, perform better on neurobehavioral tests, as compared to nerve agent-exposed subjects that were not administered the described compositions.

The disclosure is also directed to methods of treating nerve agent-induced seizures in a subject that has been exposed to a nerve agent, as described in the appended claims. In some aspects, the seizures treated are status epilepticus (SE). These methods comprising administering to the subject a pharmaceutical composition comprising an amount of dantrolene or a pharmaceutically acceptable salt thereof. As used herein, treatment of nerve agent-induced seizures results in a reduction in the severity or duration of the seizures. In other aspects, the treatment results in a reduction in both the severity and duration of the seizure.

The amount of dantrolene, or a pharmaceutically acceptable salt thereof, that is therapeutically effective to treat the subject according to any of the described methods should be determined by a practitioner skilled in the art. The therapeutically effective amount can be the amount needed to treat the subject with a single dose. Alternatively, the therapeutically effective amount can be the cumulative amount of dantrolene needed to treat the subject with more than one dose, for example multiple doses, over a chronic or prolonged course of treatment.

In the invention, the subject is a human and the therapeutically effective amount of the dantrolene is 1 mg/kg to about 30 mg/kg, administered in one or two doses. In other aspects, the therapeutically effective amount of dantrolene is 1 mg/kg to about 20 mg/kg. In other aspects, the therapeutically effective amount of dantrolene is about 5 mg/kg to about 30 mg/kg. In other aspects, the therapeutically effective amount of dantrolene is about 10 mg/kg to about 30 mg/kg. In other aspects, the therapeutically effective amount of dantrolene is about 15 mg/kg to about 30 mg/kg. In other aspects, the therapeutically effective amount of dantrolene is about 20 mg/kg to about 30 mg/kg. In other aspects, the therapeutically effective amount of dantrolene is about 5 mg/kg to about 20 mg/kg. In other aspects, the therapeutically effective amount of dantrolene is about 5 mg/kg to about 15 mg/kg. In other aspects, the therapeutically effective amount of dantrolene is about 5 mg/kg to about 10 mg/kg. In other aspects, the therapeutically effective amount of dantrolene is about 10 mg/kg to about 20 mg/kg. In other aspects, the therapeutically effective amount of dantrolene is about 2 mg/kg to about 10 mg/kg, preferably from about 2 mg/kg to about 6 mg/kg. In other aspects, the therapeutically effective amount of dantrolene is about 15 mg/kg to about 20 mg/kg. In other aspects, the therapeutically effective amount of dantrolene is about 1, 2, 3, 4, 5, 6, 7, 8, 9, 10, 11, 12, 13, 14, 15, 16, 17, 18, 19, 20, 21, 22, 23, 24, 25, 26, 27, 28, 29, or about 30 mg/kg.

In some aspects of the disclosure, the timing of the administration of the pharmaceutical composition comprising dantrolene or a pharmaceutically acceptable salt thereof, to the subject, after exposure to a nerve agent, can affect the amount of neural necrosis protection conferred to the subject.

In some aspects of the disclosure, the timing of the administration of the pharmaceutical composition comprising dantrolene or a pharmaceutically acceptable salt thereof, to the subject, after exposure to a nerve agent, can affect the amount of decrease in central nervous system function conferred to the subject.

In some aspects of the disclosure, the timing of the administration of the pharmaceutical composition comprising dantrolene or a pharmaceutically acceptable salt thereof, to the subject, after exposure to a nerve agent can affect the treatment of nerve agent-induced seizures in the subject.

Compositions comprising dantrolene, or a pharmaceutically acceptable salt thereof, can be administered chronically to the subject, in two or more doses, that is, over the course of two or more weeks, for example, 2, 3, 4, 5, 6, 7, 8 or more weeks, after the subject has been exposed to the nerve agent. Compositions comprising dantrolene, or a pharmaceutically acceptable salt thereof, can be administered acutely to the subject, in one or more doses, that is, over the course of less than two weeks, for example, over the course of hours or days, for example, over 1, 2, 3, 4, 5, 6, 7, 8, 9, 10, 11, 12, 13, 14, 15, 16, 17, 18, 19, 20, 21, 22, 23, or 24 hours or over 2, 3, 4, 5, 6, 7, 8, 9, 10, 11, 12, or 13 days, after the subject has been exposed to the nerve agent.

Regarding the timing of the pharmaceutical composition comprising dantrolene, or a pharmaceutically acceptable salt thereof, in some aspects, the pharmaceutical composition comprising dantrolene, or a pharmaceutically acceptable salt thereof, at least one dose is administered to the subject 24 hours or less after the subject has been exposed to the nerve agent. In some aspects, the pharmaceutical composition comprising dantrolene, or a pharmaceutically acceptable salt thereof, at least one dose is administered to the subject 20 hours or less after the subject has been exposed to the nerve agent. In some aspects, the pharmaceutical composition comprising dantrolene, or a pharmaceutically acceptable salt thereof, at least one dose is administered to the subject 16 hours or less after the subject has been exposed to the nerve agent. In some aspects, the pharmaceutical composition comprising dantrolene, or a pharmaceutically acceptable salt thereof, at least one dose is administered to the subject 12 hours or less after the subject has been exposed to the nerve agent. In some aspects, the pharmaceutical composition comprising dantrolene, or a pharmaceutically acceptable salt thereof, at least one dose is administered to the subject 8 hours or less after the subject has been exposed to the nerve agent. In some aspects, the pharmaceutical composition comprising dantrolene, or a pharmaceutically acceptable salt thereof, at least one dose is administered to the subject 4 hours or less after the subject has been exposed to the nerve agent. In some aspects, the pharmaceutical composition comprising dantrolene, or a pharmaceutically acceptable salt thereof, at least one dose is administered to the subject 2 hours or less after the subject has been exposed to the nerve agent. In some aspects, the pharmaceutical composition comprising dantrolene, or a pharmaceutically acceptable salt thereof, at least one dose is administered to the subject 1 hour or less after the subject has been exposed to the nerve agent. In some aspects, the pharmaceutical composition comprising dantrolene, or a pharmaceutically acceptable salt thereof, at least one dose is administered to the subject within about 1, 2, 3, 4, 5, 6, 7, 8, 9, 10, 11, 12, 13, 14, 15, 16, 17, 18, 19, 20, 21, 22, 23, or within about 24 hours after the subject has been exposed to a nerve agent.

While in some aspects, the pharmaceutical composition comprising dantrolene or a pharmaceutically acceptable salt thereof, can deliver the therapeutically effective amount of dantrolene to the nerve agent-exposed subject in one dose. In other aspects, two or more doses of the pharmaceutical composition may be needed to deliver the therapeutically effective amount of dantrolene to the nerve agent-exposed subject. For example, 2, 3, 4, 5, 6, 7, 8, 9, or 10 doses of the pharmaceutical composition may be needed to deliver the therapeutically effective amount of dantrolene to the nerve agent-exposed subject. These additional dosages can be administered substantially concurrently with the first dose. In other aspects, the additional dosages are separated in time from the first dose. In those aspects wherein 3 or more doses are administered, each dose can be separated in time from the administration of any other dose.

In some aspects of the disclosure, the administration of dantrolene to the nerve agent-exposed subject is an adjunct therapy for nerve agent exposure. Subjects exposed to a nerve agent can also be administered one or more nerve agent antidotes. One class of antidotes for nerve agent-exposure is acetylcholinesterase reactivators, for example asoxime chloride (HI-6). Another class of antidotes for nerve agent-exposure is reversible antagonists of acetylcholine receptors, for example, atropine, *e.g.,* atropine methyl nitrate. Subjects exposed to nerve agents may also be administered anti-seizure medication. Exemplary anti-seizure medications include aldehydes *(e.g.,* paraldehyde), aromatic allylic alcohols (*e.g.,* stiripentol), benzodiazepines (*e.g.,* clobazam, clonazepam, clorazepate, diazepam, midazolam, lorazepam, nitrazepam, temazepam, nimetazepam), barbiturates (*e.g.*, phenobarbital, methylphenobarbital, barbexaclone), bromides (*e.g.,* potassium bromide), carbamates (*e.g.,* felbamate), carboxamides (*e.g.,* carbamazepine, oxcarbazepine, eslicarbazepine acetate), fatty acids (*e.g*., valproic acid, sodium valproate, divalproex sodium, vigabatrin, progabide, tiagabine), topiramate, GABA analogs (*e.g*., gabapentin, pregabalin), hydantoins (*e.g.*, ethotoin, phenytoin, mephenytoin, fosphenytoin), oxazolidinediones (*e.g.,* paramethadione, trimethadione, ethadione), propionates (*e.g.,* beclamide), pyrimidinediones (*e.g.,* primidone), pyrrolidines (*e.g.,* brivaracetam, levitiracetam, seletracetam), succinimides (*e.g.,* ethosuximide, phensuximide, mesuximide), sulfonamides (*e.g.,* acetazolamide, sultiame, methazolamide, zonisamide), triazines (*e.g.,* lamotrigine), ureas (*e.g.,* pheneturide, phenacemide), valproylamides (*e.g*., valpromide, valnoctamide), perampanel, and combinations thereof. In some aspects, the anti-seizure medication is a benzodiazepine, for example, midazolam. In other aspects, the anti-seizure medication is a barbiturate. In still other aspects, the anti-seizure medication is a hydantoin. In some aspects, the anti-seizure medication is paraldehyde. In other aspects, the anti-seizure medication is potassium bromide. In some aspects, the anti-seizure medication is a fatty acid. In other aspects, the anti-seizure medication is topiramate.

In those aspects wherein the nerve agent-exposed subject is administered an antidote, the dantrolene is administered after the antidote has been administered. For example, the dantrolene can be administered after the administration of the acetylcholinesterase reactivator and/or after the administration of the reverse antagonist of acetylcholine receptors.

In those aspects wherein the nerve agent-exposed subject is administered an anti-seizure medication, the dantrolene can be administered concurrently with the administration of the anti-seizure medication. The dantrolene can be administered substantially currently with the administration of the anti-seizure medication, as well, for example, within about 5 minutes of the anti-seizure medication administration. In other embodiments, the dantrolene is administered before the anti-seizure medication is administered. In other embodiments, the dantrolene is administered after the anti-seizure medication is administered.

The pharmaceutical composition comprising dantrolene, or a pharmaceutically acceptable salt thereof can be administered intravenously. In other aspects, the pharmaceutical composition comprising dantrolene, or a pharmaceutically acceptable salt thereof can be administered transdermally. In other aspects, the pharmaceutical composition comprising dantrolene, or a pharmaceutically acceptable salt thereof can be administered intramuscularly. In other aspects, the pharmaceutical composition comprising dantrolene, or a pharmaceutically acceptable salt thereof can be administered intraosseously. In other aspects, the pharmaceutical composition comprising dantrolene, or a pharmaceutically acceptable salt thereof can be administered subcutaneously.

The pharmaceutical compositions of the invention comprise dantrolene or a pharmaceutically acceptable salt thereof, mannitol, a polysorbate (*e.g.*, polysorbate 80), a povidone (e.g. povidone K12), an optional pH adjustor (e.g. NaOH or HCl), and water.

A particularly preferred pharmaceutical composition comprising dantrolene or a pharmaceutically acceptable salt thereof is RYANODEX^{®} (dantrolene sodium, Eagle Pharmaceuticals, Woodcliff Lake, NJ). RYANODEX^{®} is an injectable suspension provided as a sterile lyophilized powder. It is supplied in 20 mL vials containing 250 mg dantrolene sodium, 125 mg mannitol, 25 mg polysorbate 80, 4 mg povidone K12, and sufficient sodium hydroxide or hydrochloric acid for pH adjustment. When reconstituted with 5 mL sterile water for injection USP, this yields a suspension.

The following examples are provided to illustrate some of the concepts described within this disclosure. While each example is considered to provide specific individual embodiments of disclosure, none of the Examples should be considered to limit the more general embodiments described herein. In the following examples, efforts have been made to ensure accuracy with respect to numbers used (e.g. amounts, temperature, etc.) but some experimental error and deviation should be accounted for.

### EXAMPLES

Examples dealing with the fronto-parietal cortex protection form part of the present invention. Examples dealing with other brain regions represent reference examples.

### Example 1

### Study Overview

The study object was to determine whether RYANODEX^{®} has neuroprotective effects in an established GD (soman) survival model in rats. Single doses of 10 or 30 mg/kg RYANODEX^{®} were given by bolus IV injection at 20 or 50 minutes following the onset of soman-induced seizures. Survival was facilitated by treatment with asoxime chloride (HI-6) thirty minutes before subcutaneous (SQ) soman injection, atropine methyl nitrate one minute after SQ soman injection, and midazolam twenty minutes after the onset of soman-induced seizures that attain a Racine score of at least 3. Controls included one group of untreated (naive) rats and another group that will receive sterile water 50 minutes after the onset of soman-induced seizures.

A series of neurobehavioral tests were carried out over a period of approximately 28 days following single-dose GD exposure. On day 29, all rats were sacrificed under anesthesia via exsanguination and intracardiac perfusion. Brain was collected from each rat for microscopic neuropathology examination, and heart was collected from each rat for possible pathology examination.

Experimental subjects were jugular vein cannulated > 8 week old male Sprague-Dawley rats (Charles River Laboratories) weighing 300-500 g on the day of exposure. The Sucrose Preference Test and Forced Swim neurobehavioral tests were used to evaluate potential deficits in tasks involving learning and/or sensory motor integration.

### Materials

**Soman** (GD)- diluted with 0.9% Sodium Chloride. Soman is an organophosphorus nerve agent that deactivates acetylcholine esterase (AChE) by forming an adduct with the enzyme.

| | | |
|---|---|---|
| • | Chemical Name: | Pinacolyl methyl phosphonofluoridate |
| • | Formula: | C₇H₁₆FO₂P |
| • | Molecular Weight: | 182.17 |
| • | MRIGlobal Lot#: | GD090415-DOC-1 |
| • | Primary Standard ID: | 13972-49-3 |
| • | Purity: | 100% |
| • | Storage Conditions: | < 4 °C |

**HI-6:** Chemical Name: [(E)-[1-[(4-carbamoylpyridin-1-ium-1-yl)methoxymethyl]pyridin-2-ylidene]methyl]-oxoazanium;methanesulfonate (asoxime chloride) 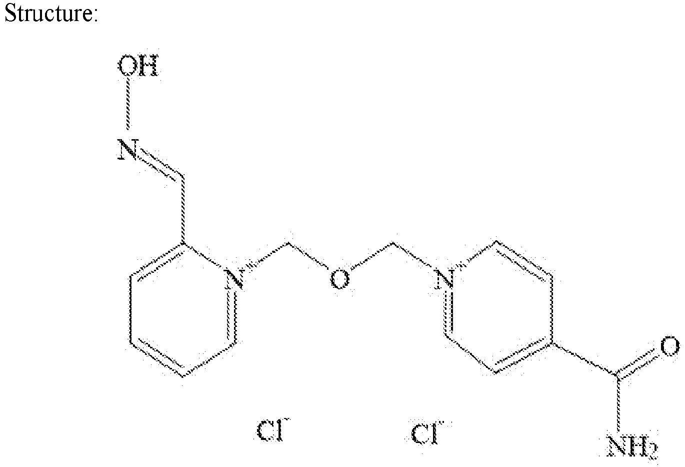
Formula: C₁₄H₁₆Cl₂N₄O₃
Molecular Weight: 359.207

**Atropine methyl nitrate:** Chemical Name: (8,8-dimethyl-8-azoniabicyclo[3.2.1]octan-3-yl) 3-hydroxy-2-phenylpropanoate;nitrate 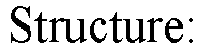 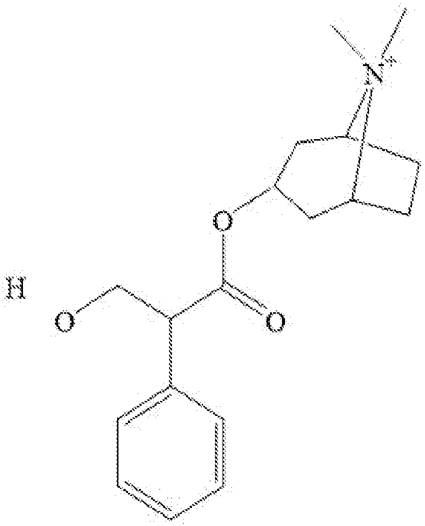
Formula: C₁₈H₂₆N₂O₆
Molecular Weight: 366.414

**Midazolam:** Chemical Name: 8-chloro-6-(2-fluorophenyl)-1-methyl-4H-imidazo[1,5-a][1,4]benzodiazepin 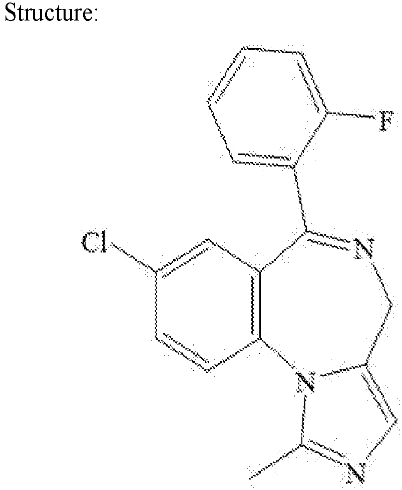
Formula: C₁₈H₁₃ClFN₃
Molecular Weight: 325.771

**RYANODEX^{®}** is an FDA-approved drug for treatment of malignant hyperthermia in conjunction with appropriate supportive measures and for prevention of malignant hyperthermia in patients at high risk.

### Animals

For this study, 48 male Sprague Dawley rats (plus 8 extras/replacements for a total of 56; Charles River Laboratories), > 8 weeks old and 300-500 g with jugular vascular catheters implanted by the vendor were used in the study. Rats were identified by Tail Marking or Ear Tag bearing a unique alphanumerical designation. (See SOP MRI-1504, "Procedure for Identification of Animals and Cages").

All rats included in the study were in good health, free from any signs of clinical disease, and had patent catheters suitable for dosing at study start. Upon delivery, animals were inspected for signs of ill-health and quarantined in the Spencer conventional animal facility for no less than 48 h. A veterinarian examined the health of the animals and released them for the study (see SOP MRI-1501, "Acclimation and / or Quarantine Procedures for Animals"). In accordance with SOP MRI-1500, the animals were weighed within three days of delivery. During the period from the day after animal arrival through the entire study, rats were handled by staff members at least twice daily for acclimation and consistency throughout the study.

All rats were provided with Lab Diet Certified Rodent Food, identified as to source, lot number, and Certificate of Analysis, *ad libitum.* No contaminants which could affect the results of the study were present in the feed, as verified by the certificate of analysis (for each lot, provided by the vendor; see SOP MRI-1510, "Procedure for Feeding Animals"). Water bottles (one water bottle/cage) were provided with tap water throughout the study with the exception of the period of acclimation and conduct of Sucrose Preference Test (See *infra*), during which rats were provided with two water bottles containing tap water or one water bottle containing tap water and one water bottle containing a 1% sucrose solution.

Animals were housed in polycarbonate Tecniplast caging with wire top (Tecniplast, Phoenixville, PA) throughout the acclimation/quarantine and in-life period. Animals were singly housed to prevent damage to the catheters. The rats were housed in environmentally controlled rooms, with at least 10 air changes per hour. The rooms were maintained at a temperature of 68.0 °F to 79.0 °F and a relative humidity of 50% ± 20% with a 12-h light/dark cycle per day. Room temperature, humidity, and light cycles (12 hour: 12 hour reversed light / dark) were monitored by the AmegaView monitoring system 24 h/day or by a Hygrothermograph during the study (see SOP MRI-1170, "Temperature and Humidity Monitoring in Animal Rooms").

### Doses

**HI-6, soman, atropine methyl nitrate, and midazolam:** Single doses of HI-6 (IP, 125 mg/kg); soman (SC, 154 µg/kg, 1.4 x LD₅₀), atropine methyl nitrate (IM, 2 mg/kg); and midazolam (IM, 2 mg/kg) were selected based on previous experience with the model. This regimen was expected to cause convulsions that achieve a Racine score of at least 3 and an acceptable number of survivors for follow-up study.

**RYANODEX^{®}:** In a single-dose tolerability evaluation in rats, IV bolus doses of 12, 40, and 60 mg/kg RYANODEX^{®}were given to separate groups of jugular vein cannulated rats. Twelve mg/kg was well tolerated. A dose of 40 mg/kg resulted in slight body weight loss two days following dosing, and 60 mg/kg resulted in moribund sacrifice approximately twenty-four hours after dosing for all five rats that received this dose. Single IV bolus doses of 10 and 30 mg/kg RYANODEX^{®} were selected for this study based on their expected tolerability. RYANODEX^{®} doses of 10 and 30 mg/kg in rat are equivalent to human doses of approximately 2 and 6 mg/kg, respectively.

### Dosing Scheme

A total of 48 plus up to 8 extra male Sprague-Dawley rats with jugular catheters implanted by the vendor were used in 6 groups as shown in Table 1.

**Table 1. Design for Preliminary Efficacy Study with RYANODEX^{®}**

| Group | Pretreatment HI-6 (IP, 30 min prior to GD challenge) | Soman "GD" (SQ) | Atropine Methyl Nitrate (IM, 1 min post GD) | Midazolam (IM, ~20 minutes post-onset seizure activity) | RYANODEX^{®} IV at 20 or 50 min post-onset of seizures |
|---|---|---|---|---|---|
| A (n=8) | 125 mg/kg | 154 µg/kg (1.4 x LD₅₀) | 2.0 mg/kg | 2.0 mg/kg | 50 min with 10 mg/kg |
| B (n=8) | 125 mg/kg | 154 µg/kg (1.4 x LD₅₀) | 2.0 mg/kg | 2.0 mg/kg | 50 min with 30 mg/kg |
| C (n=8) | 125 mg/kg | 154 µg/kg (1.4 x LD₅₀) | 2.0 mg/kg | 2.0 mg/kg | 20 min with 10 mg/kg |
| D (n=8) | 125 mg/kg | 154 µg/kg (1.4 x LD₅₀) | 2.0 mg/kg | 2.0 mg/kg | 20 min with 30 mg/kg |
| E (n=8) | 125 mg/kg | 154 µg/kg (1.4 x LD₅₀) | 2.0 mg/kg | 2.0 mg/kg | Sterile water control at 50 min |
| F (N=8) | 0 mg/kg | 0 µg/kg | 0 mg/kg | 0 mg/kg | NA |

| | | | | | |
|---|---|---|---|---|---|
| Note: for groups C and D, RYANODEX^{®} will be given right after Midazolam injection. | | | | | |

Dose administration started with Group D, followed by Groups B, A, C, E, and F, respectively.

### Dose Preparation

GD was prepared in ice cold 0.9% Sodium Chloride according to SOP MRI-5821 "Preparation of Standards and Samples from Research Development and Testing Evaluation (RDTE) Dilute Solutions".

RYANODEX^{®} was prepared by adding 5 mL of sterile water for injection to one vial of the lyophilized product.

### Experimental Design

### Animal Preparation

Prior to or on Study Day 1 (day of GD exposure), rats were transferred to the dilute animal laboratory complex.

### Dosing

On Study Day 1, all animals in Groups A - E received the cholinesterase reactivator, HI-6 (125 mg/kg) via the intraperitoneal (IP) route 30 minutes prior to GD challenge. All rats in Groups A-E were exposed to GD (154 µg/kg or ~1.4 x LD₅₀) by a single subcutaneous injection. Approximately one minute post-GD injection for Groups A-E, atropine methyl nitrate (AMN) was administered via IM route (2.0 mg/kg). Approximately 20 minutes post-onset of seizure activity following GD exposure as defined in Table 1 above, all animals in Groups A-E, received midazolam at a dose of 2 mg/kg intramuscularly. Group F received no HI-6, no GD, no AMN, no Midazolam, and no RYANODEX^{®}. RYANODEX^{®} was administered via a Hamilton microsyringe, then the catheter flushed with 0.2 mL of sterile water for injection to ensure complete delivery to the vein.

Initiation of seizure activity was defined by a score of "3" on the Racine scale as described below:
1= immobilization & staring
2= head-nodding, "wet dog shakes"
3= forelimb clonus
4= bilateral forelimb clonus
5= bilateral forelimb clonus, rearing and loss of balance

If a rat did not achieve this score of "3" after approximately 20 minutes following GD exposure, it was replaced in the study.

As shown in Table 1, rats in Groups A and C received a 10 mg/kg dose of RYANODEX^{®} IV and rats in Groups B and D received a 30 mg/kg dose of RYANODEX^{®} IV either at 50 or 20 min post-onset of seizures (Racine scale of "3"). Group E rats received a dose of control (sterile water for injection) IV at a volume comparable to the highest dose of RYANODEX^{®} at 50 min post-onset of seizures.

At approximately 6 hours post-GD challenge for Groups A-E, and then every AM and PM, rats were administered 5 mL of Lactate Ringers Solution SQ for up to the first 7 days post-challenge to aid recovery. Once animals recovered from challenge as indicated by observing them eating and drinking freely, moving about the cage freely, and not having any signs of dehydration (no tenting of skin on back), the SQ fluids ceased. This was in part at the discretion of the veterinarian based on his/her assessment of the animals. Furthermore, post-challenge during this same time frame (up to 7-day post-challenge), the animals were offered hydrogels (ClearH₂O) and gruel (moistened feed in crock on cage floor) to help them recover from the effects of the GD challenge.

### Body Weights

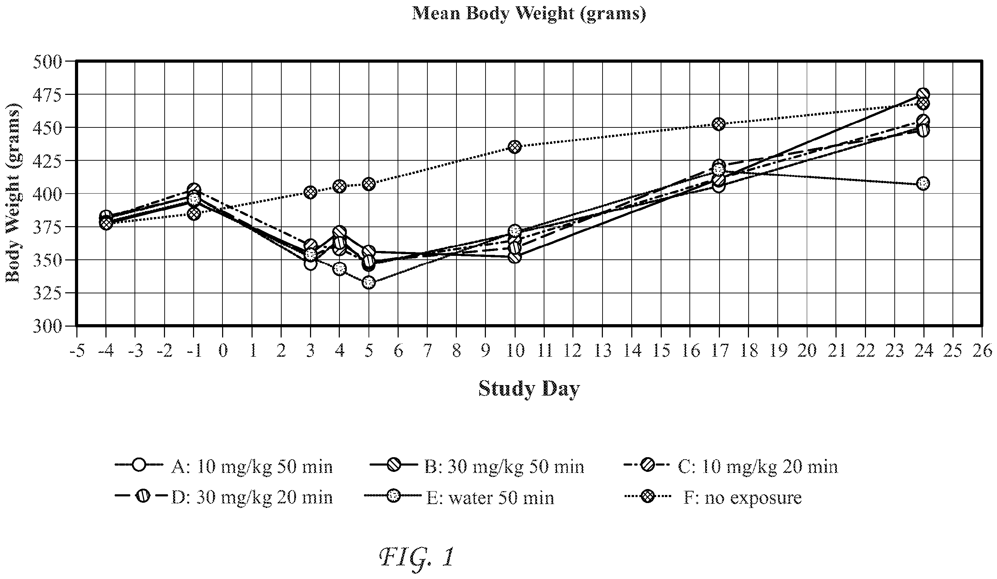

Body weights were recorded for each animal on study within the first 72 h of receipt from the vendor, then again on Study Days 1, 3, 5, 10, 17, 24, and 28. More frequent body weights were collected if the Study Director discerned that the animals continued to lose weight post-challenge. If any animal lost more than 10% of its original body weight, it was weighed on a daily basis until the animal was consistently gaining rather than losing weight. Body weights of all groups is depicted in Figure 1. Body weights of all soman-exposed groups treated with RYANODEX^{®} (Groups A, B, C, D) recovered to negative control (Group F) values by study day 25.

### Clinical Observations

General health observations were performed daily through the quarantine and acclimation period. Animals were handled twice daily throughout the quarantine and acclimation period and through the entire in life. On Study Day 1, the rats were observed prior to exposure. After GD exposure, the rats were monitored frequently to assess the initial appearance of seizures. After midazolam, RYANODEX^{®} and/or sterile water dosing, the animals were observed a minimum of twice daily, and at least once on the day of euthanasia. Clinical observations including hunched posture, dehydration, rough coat and inappetence were evaluated and recorded. The modified Racine score was recorded at each observation point if applicable. Any other abnormal clinical observations were also recorded at this time in the study notebook or appropriate data capture form as per SOP MRI-1528 "Procedure for Observation of Animals". Time of death was documented; any animals found dead were documented at the time they were found as such.

### Animal Disposition

Day 1 of the study was the day of soman exposure and RYANODEX^{®} treatment. All rats that died were found dead (not euthanized in a moribund condition). The protocol-specified number of rats was 8 survivors/group, which was not achieved due to mortality associated with high soman dose. The overall survival was 27 out of 45 rats exposed to soman (60%). See table 2.

**Table 2**

| **Group** | **Regimen** | **Study Day of Death** | **Mortality/Total (%)** | **No. Rats that Completed Study** |
|---|---|---|---|---|
| A | 10 mg/kg RYANODEX^{®} at 50 min | 1 (3 rats) ^{(a)}, 2, 15 | 5/10 (50%) | 5 |
| B | 30 mg/kg RYANODEX^{®} at 50 min | 1,2,3,8 | 4/9 (44.4%) | 5 |
| C | 10 mg/kg RYANODEX^{®} at 20 min | 1, 15 | 2/8 (25%) | 6 |
| D | 30 mg/kg RYANODEX^{®} at20 min | 1, 6, 11 | 3/9 (33%) | 6 |
| E | Water at 50 min | 1 ^{(b)}, 2, 22, 23 | 4/9 (44.4%) | 5 |
| F | No exposure / No treatment | Not applicable | 0/8 (0%) | 8 |

(a) One Group A rat was found dead before receiving midazolam and RYANODEX^{®}.
(b) One Group E rat was found dead before receiving water.

### Characteristics of the Model

| | |
|---|---|
| Survival: | 27 of 45 rats exposed to soman (60%) |
| Time to seizure onset following soman injection: | 6.8 minutes ± 1.93 |
| | Range: 4 - 11 minutes |
| Time to maximum Racine score ^{(a)} following soman injection: | 8.4 minutes ± 2.99 |
| | Range: 4 - 17 minutes |
| Maximum Racine score achieved on Day 1: | 4.2 ± 0.59 |
| | Range: 3 - 5 |
| Clinical observations of seizures during 28-day in-life phase: | Although rats were not observed continuously, isolated Racine scale 5 seizures were seen in individual animals throughout the in-life phase. |
| Body Weight during 28-day in-life phase: | 15% body weight loss occurred for all groups following soman exposure; recovery to control levels by Study Day 25, EXCEPT for positive control animals. |

### Neurobehavioral Tests

Brain areas damaged by soman exposure included the hippocampus and entorhinal, frontal, and parietal cortices. These areas contain structures and neural circuits for learning, memory formation, information processing, and other cognitive processes. To evaluate the potential neuroprotective effects of RYANODEX^{®}, rats were evaluated using a series of established behavioral tests that require learning, memory, sensory motor integration, and adaptive responses.

The tests selected were: 1) Sucrose Preference Test and 2) Forced Swim Test.

### Sucrose Preference Test

The Sucrose Preference Test (SPT) utilizes the natural inclination of rats to prefer sugar water over regular water. It is an established test to measure pleasure seeking behavior (hedonia) or lack of it (anhedonia) and requires animals to adapt to change in left vs. right placement of bottles containing tap water and 1% sucrose water. The SPT occurred on Study Days 14 and 15.

Rats were housed individually with *ad libitum* access to food and water (single water bottle in each cage) before the SPT. For the acclimation portion of the SPT, 2 water bottles were introduced into each rat's home cage for 5 - 6 days. The water bottles were fitted with sipper tubes that minimize leakage and were weighed approximately every 24 hours. Following the acclimation phase, one water bottle was filled with approximately 200 mL of 1% sucrose solution, and the other water bottle with approximately 200 mL of tap water. Twenty-four hours later, the amount of fluid remaining in each bottle was recorded. The L/R placement of the bottles was then switched, and the amount of fluid remaining in each bottle was again recorded twenty-four hours later. The amount (mL) of sucrose solution consumed was expressed as a percent of the total volume of fluid consumed (sucrose water plus water) over each of the two 24-hour periods and compared across groups and days.

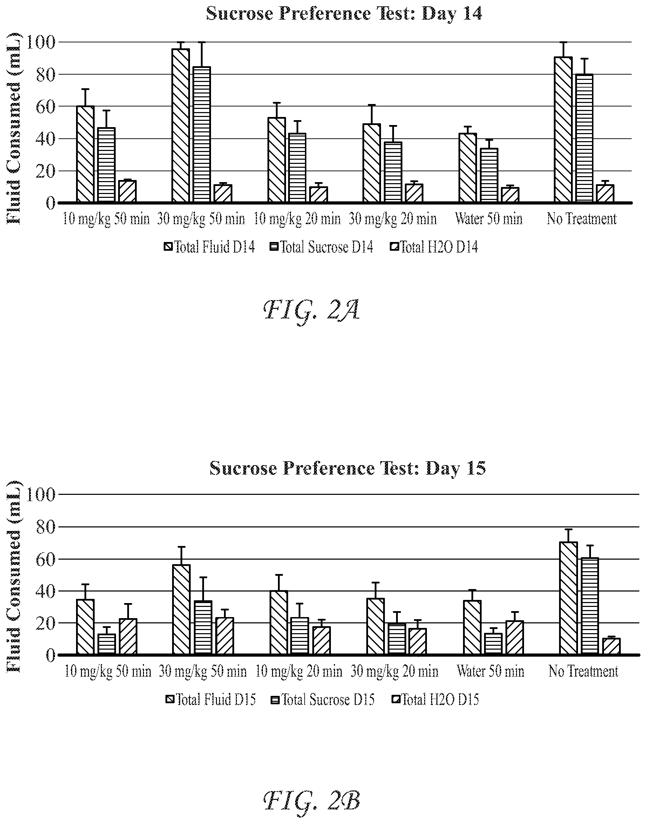

SPT results are depicted in Figures 2A and 2B. On the second day of the test (Day 15), the positive control group (water at 50 minutes) did not maintain preference for sucrose, in contrast to 3 of 4 groups that received RYANODEX^{®}.

### Forced Swim Test

The Forced Swim Test (FST) was developed in the late 1970s by Porsolt as a quick way to screen for efficacy of antidepressant drugs in rodents. The increased immobility that occurs towards the end of the 5-minute FST in untreated ("normal") rodents was interpreted to reflect "behavioral despair", and it's reversal with antidepressant drugs correlated with the antidepressant efficacy of these agents in people. However, the construct validity of this test has come under question for many reasons, including: 1) acute effects of antidepressants are tested in FST whereas in clinically depressed patients, the drugs require 4 - 6 weeks for clinical improvement; 2) the dependent variable in the FST is the animal's acute response to the test and not a characteristic of the animal; and 3) the interpretation of floating behavior as 'behavioral despair' is anthropomorphic. It is now believed that the progressive immobility seen in untreated rats reflects an adaptive response to the acute stress of being placed in a container with no possibility of escape.

In the FST, swimming activity and immobility were measured in a glass cylindrical chamber (46cm H x 30cm D) filled with water (30 cm height, 25°C). Thermometers were used to ensure that the water temperature is a constant 24-26°C for all animals. Two swimming sessions were carried out, one as an initial 15 min 'pre-test,' followed 24 hours later by a second 5 min 'test.' Test sessions were video recorded. Time spent actively swimming and time spent immobile was scored for each minute of the FST.

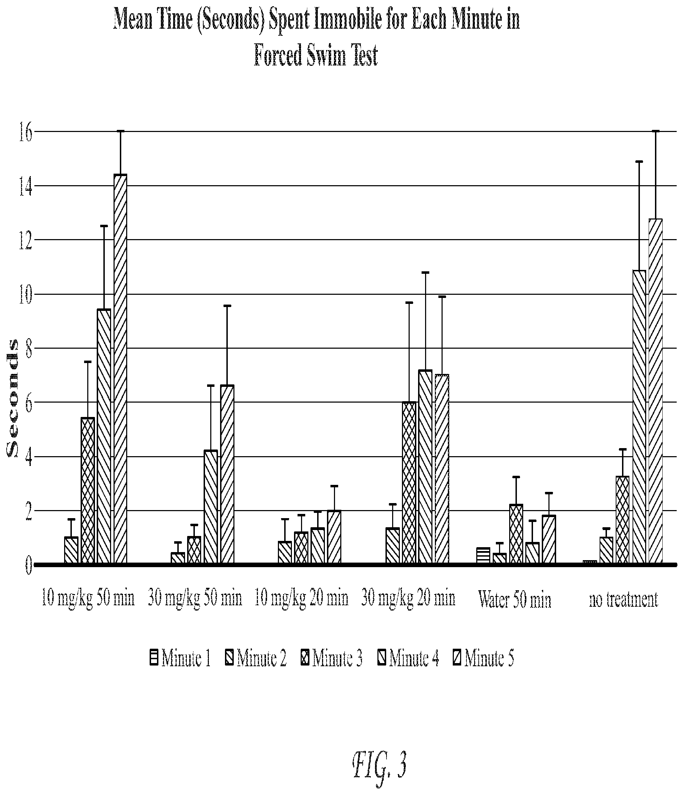

FST was conducted on Study Days 25 and 26. On Study Day 25, each rat was placed in the water-filled plexiglass cylinder for 15 minutes. On Study Day 26, each rat was placed back in the water-filled plexiglass cylinder for the 5 minute test. Time spent moving and immobile were scored for each rat during each minute of the test. FST results are depicted in Figure 3. RYANODEX^{®}-treated groups displayed similar trend as negative control (no treatment) group, that is, progressive increase in time spent immobile over 5 minute test duration.

### Order of Neurobehavioral Tests

The order of the neurobehavioral tests proceeded from least stressful to most stressful. No two tests were performed on the same day. All testing occurred in the morning. The tests were run as follows (after Study Day 1 as day of challenge):
∘ On Study Day 8, Sucrose Preference Test (lasts up to 8 days)
∘ On Study Day 25, Forced Swim Test (lasts 2 days)

The order of animals tested was randomized, and the group identities of the rats undergoing both tests was masked such that the technicians running these tests was blind to treatment group. Scoring of results from testing was done using the videotapes.

### Euthanasia and Tissue Harvest and Processing

For each group on Study Day 29, all surviving rats were anesthetized via an IP injection of target doses of ≤ 200 mg/kg ketamine and ≤ 20 mg/kg xylazine. Rats were perfused transcardially with heparinized saline followed by 0.4% paraformaldehyde (perfusion protocol follows Rao, et al (2006). The heart of each rat was collected and stored in fixative for possible evaluation.

Following tissue collection, animal carcasses were disposed of according to SOP MRI-1526 "Disposal of Animal Carcasses and Medical Waste in the Spencer Animal Care Facility".

The brains remained in paraformaldehyde fixative overnight at 4°C for at least 4 days and then shipped in their individual vials containing fixative on ice packs overnight to HSRL, Inc. for evaluation. Each brain was uniformly trimmed into 7 sections as described in Rao, et al (2014) and Bolon, et al (2013) to obtain maximum anatomic presentation of the entire brain, including areas known to be affected by soman and other organophosphorus nerve agents (eg, piriform cortex, entorhinal cortex, hippocampus, amygdala, thalamus, cerebellum). The sections were routinely processed, sectioned at 5 microns, embedded in paraffin, and stained with hematoxylin and eosin. Personnel were blinded to dose groups and treatment.

### Neuropathology

The 7 brain sections from each rat were evaluated microscopically using a 6-point semi-quantitative scoring system. Microscopic lesions were graded on a 6-point scale:
0 = normal
1 = 1 - 5 cells affected per 40X microscopic field
2 = 6 - 20 cells affected per 40X microscopic field
3 = 21 - 50 cells affected per 40X microscopic field
4 = 50% - 80% of cells affected per 40X microscopic field
5 = >80% of cells affected per 40X microscopic field

Table 3 set for pathology data from the fronto-parietal cortices, with showing the proportion of animals per necrosis score by group.

**Table 3**

| **Necrosis Score** | | | | | | |
|---|---|---|---|---|---|---|
| | **0** | **1** | **2** | **3** | **4** | **5** |
| **Study Group** | **Normal** | **1-5% Necrosis** | **6-20% Necrosis** | **21-50% Necrosis** | **51-80% Necrosis** | **>80% Necrosis** |
| A: 10 mg/kg RYANODEX^{®} 50 min | 20 | 40 | 20 | 20 | 0 | 0 |
| B: 30 mg/kg RYANODEX^{®} 50 min | 0 | 60 | 40 | 0 | 0 | 0 |
| C: 10 mg/kg RYANODEX^{®} 20 min | 34 | 50 | 0 | 17 | 0 | 0 |
| D: 30 mg/kg RYANODEX^{®} 20 min | 50 | 34 | 17 | 0 | 0 | 0 |
| E: Water 50 min | 20 | 0 | 0 | 60 | 20 | 0 |
| F: no challenge | 100 | 0 | 0 | 0 | 0 | 0 |

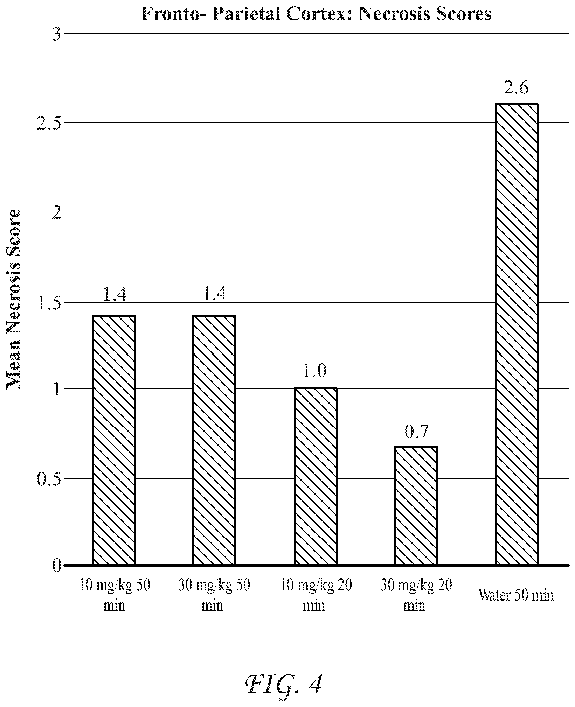

Mean necrosis scores from the fronto-parietal cortex are depicted in Figure 4.

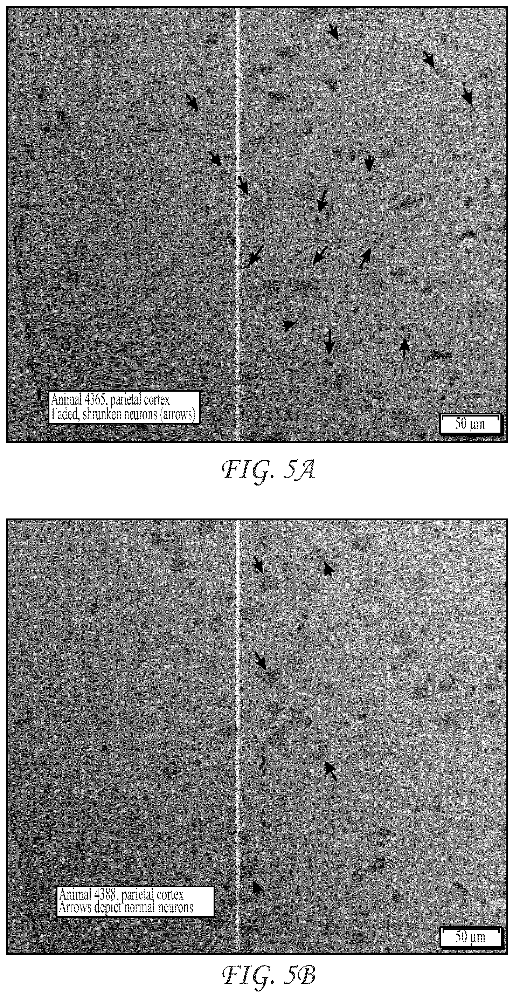

Photomicrographs are depicted in Figures 5A and 5B. Figure 5A depicts a photomicrograph of an animal from Group E, positive control, water 50 minutes post SE onset. The arrows mark faded, shrunken neurons. This animal received a necrosis score of 4. Figure 5B depicts a photomicrograph of an animal from Group D, 30 mg/kg RYANODEX^{®} 20 minutes post SE onset. The arrows mark normal neurons. This animal received a necrosis score of 0.

Table 4 depicts microscope pathology findings. Group F animals (untreated) had no significant findings. Group E (positive control) had expected findings. The reduced necrosis scope for fronto-parietal cortex for RYANODEX^{®}-treated rats, as compared with positive control (Group E) was consistent with literature reports.

**Table 4**

| **Brain Location** | **Mean (SD) Pathology Score for Necrosis** | | | | |
|---|---|---|---|---|---|
| | **A 10 mg/kg at 50 min** | **B 30 mg/kg at 50 min** | **C 10 mg/kg at 20 min** | **D 30 mg/kg at 20 min** | **E Water at 50 min** |
| Hippocampus, CA3 | 1.4 (0.89) | 1.0 (0.71) | 0.8 (0.75) | 1.8 (1.72) | 0.8 (0.45) |
| Hippocampus, CA2 | 4.8 (0.45) | 4.2 (1.79) | 3.5 (1.97) | 4.7 (0.82) | 5.0 (0.00) |
| Hippocampus, CA1 | 4.4 (1.34) | 4.8 (0.45) | 3.7 (2.16) | 4.7 (0.82) | 5.0 (0.00) |
| Entorhinal Cortex | 4.0 (1.73) | 3.6 (2.19) | 4.0 (1.10) | 3.2 (2.48) | 3.6 (1.14) |
| Front-parietal Cortex | 1.4 (1.14) | 1.4 (0.55) | 1.0 (1.10) | 0.7 (0.82) | 2.6 (1.52) |
| Piriform Cortex | 0.4 (0.89) | 0.8 (1.30) | 0.8 (0.98) | 0.3 (0.82) | 0.4 (0.89) |
| Thalamus | 3.4 (1.69) | 3.0 (0.00) | 2.3 (1.03) | 3.7 (0.52) | 4.2 (0.04) |

### Conclusions

Overall, RYANODEX^{®}-treated animals had better performance in neurobehavioral tests, compared to non-treated animals. RYANODEX^{®}-treated animals showed lower level of cell necrosis in brain cortex, compared to non-treated animals. RYANODEX^{®}-treated animals showed a dose-dependent and time-dependent treatment effect impacting cell death. These neurobehavioral tests and neuropathology finding are consistent and appear to be directly correlated. RYANODEX^{®} was well tolerated across all groups and no new safety signals were observed.

### Example 2

### Study Overview

The study object is to determine whether RYANODEX^{®} has neuroprotective effects in an established GD (soman) survival model in rats. Single doses of 10 or 30 mg/kg RYANODEX^{®} will be given by bolus IV injection at 20 or 60 minutes following the onset of soman-induced seizures. Survival will be facilitated by treatment with asoxime chloride (HI-6) thirty minutes before subcutaneous (SQ) soman injection, atropine methyl nitrate one minute after SQ soman injection, and atropine sulfate, praldoxime chloride (2-PAM-Cl), and midazolam twenty minutes after the onset of soman-induced seizures that attain a Racine score of at least 3. Controls will include two separate groups: one group of untreated (naive) rats (negative control) and another group that will receive either sterile water or sterile water plus mannitol vehicle, 60 minutes after the onset of soman-induced seizures.

One day 2, all rats will be sacrificed under anesthesia via exsanguination and intracardiac perfusion. The brain will be collected from each rat for microscopic neuropathology examination, and the heart will be collected from each rat for possible pathology examination.

Experimental subjects will be jugular vein cannulated > 8 week old male Sprague-Dawley rats (Charles River Laboratories) weighing 300-500 g on the day of exposure.

### Materials

**Soman** (GD)- diluted with 0.9% Sodium Chloride. Soman is an organophosphorus nerve agent that deactivates acetylcholine esterase (AChE) by forming an adduct with the enzyme.

| | | |
|---|---|---|
| • | Chemical Name: | Pinacolyl methyl phosphonofluoridate |
| • | Formula: | C₇H₁₆FO₂P |
| • | Molecular Weight: | 182.17 |
| • | MRIGlobal Lot#: | GD090415-DOC-1 |
| • | Primary Standard ID: | 13972-49-3 |
| • | Purity: | 100% |
| • | Storage Conditions: | < 4 °C |

Treatments to promote survival in this model will be 1) asoxime chloride (HI-6); 2) stropine methyl nitrate; 3) stropine sulfate; 4) praldoxime chloride; 5) midazolam. HI-6 is an AChE reactivator that can displace organophosphates like soman from acetylcholinesterase (AChE) and thereby reactivate the enzyme. Atropine sulfate is a centrally acting competitive and reversible antagonist of muscarinic acetylcholine receptors; atropine methyl nitrate is a peripherally acting competitive and reversible antagonist of muscarinic acetylcholine receptors; both are used clinically to counteract the acute cholinergic syndrome induced by soman and other organophosphate nerve agents. Midazolam is a benzodiazepine for treatment of seizures. These agents are selected because oximes, atropine, and benzodiazepines represent the accepted clinical treatment for acute nerve agent / organophosphate toxicity. A three-drug regimen consisting of pralidoxime (an AChE reactivator), atropine, and diazepam (a benzodiazepine) is a currently FDA-approved countermeasure for military personnel.

**HI-6:** Chemical Name: [(E)-[1-[(4-carbamoylpyridin-1-ium-1-yl)methoxymethyl]pyridin-2-ylidene]methyl]-oxoazanium;methanesulfonate (asoxime chloride) 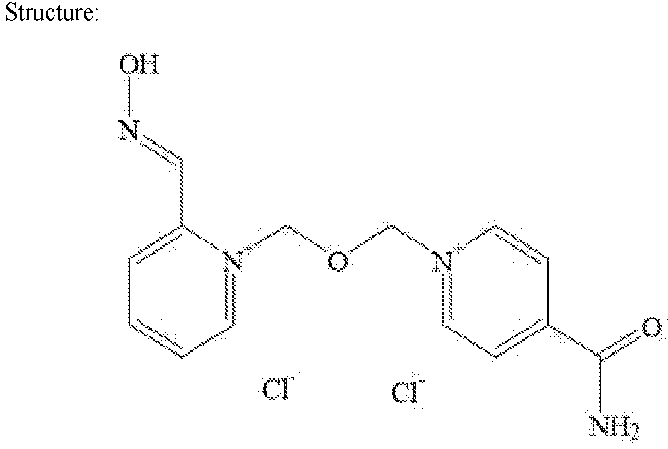
Formula: C₁₄H₁₆Cl₂N₄O₃
Molecular Weight: 359.207

**Atropine methyl nitrate:** Chemical Name: (8,8-dimethyl-8-azoniabicyclo[3.2.1]octan-3-yl) 3-hydroxy-2-phenylpropanoate;nitrate 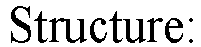 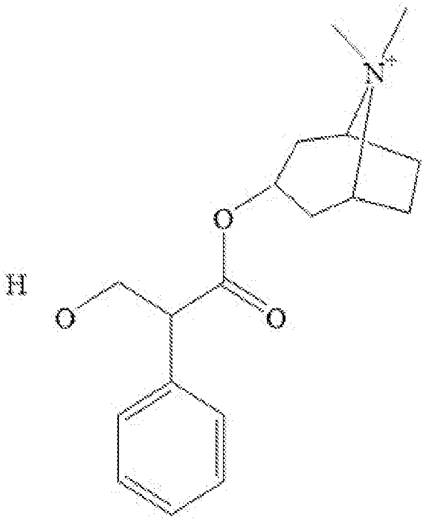
Formula: C₁₈H₂₆N₂O₆
Molecular Weight: 366.414

**Midazolam:** Chemical Name: 8-chloro-6-(2-fluorophenyl)-1-methyl-4H-imidazo[1,5-a][1,4]benzodiazepin 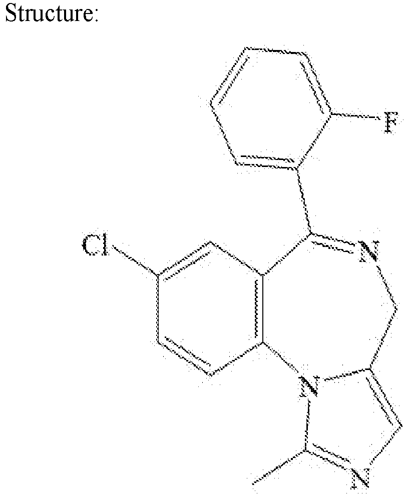
Formula: C₁₈H₁₃ClFN₃
Molecular Weight: 325.771

**RYANODEX^{®}** (dantrolene sodium) is an FDA-approved drug for treatment of malignant hyperthermia in conjunction with appropriate supportive measures and for prevention of malignant hyperthermia in patients at high risk.

**Dantrolene Sodium:** Chemical Name: hydrate of 1-[[[5-(4-nitrophenyl)-2-furanyl]methylene]amino]-2,4-imidazolidinedione sodium salt 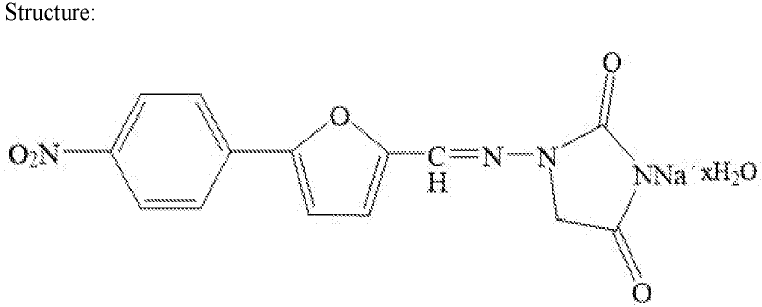 Molecular Weight: 399

Excipients: Mannitol (25 mg/mL) polysorbate 80 (5 mg/mL); povidone K12 (0.8 mg/mL)
**5% Mannitol Solution:** 5% Mannitol Solution (in Sterile Water)

Formula: C₆H₁₄O₆

### Animals

For this study, 120 male Sprague Dawley rats (plus 20 extras/replacements for a total of 140; Charles River Laboratories), > 8 weeks old and 300-500 g with jugular vascular catheters implanted by the vendor will be used in the study. Rats will be identified by Tail Marking or Ear Tag bearing a unique alphanumerical designation. (See, e.g. SOP MRI-1504, "Procedure for Identification of Animals and Cages").

All rats included in the study will be in good health, free from any signs of clinical disease, and will have patent catheters suitable for dosing at study start. Upon delivery, animals will be inspected for signs of ill-health and will be quarantined in a conventional animal facility for no less than 48 h. A veterinarian will examine the health of the animals and will release them for the study (see, e.g. SOP MRI-1501, "Acclimation and / or Quarantine Procedures for Animals"). The animals will be weighed within three days of delivery. During the period from the day after animal arrival through the entire study, rats will be handled by staff members at least twice daily for acclimation and consistency throughout the study.

All rats will be provided with Lab Diet Certified Rodent Food, identified as to source, lot number, and Certificate of Analysis, *ad libitum.* No contaminants which could affect the results of the study will be present in the feed, as verified by the certificate of analysis (for each lot, provided by the vendor; see, e.g.SOP MRI-1510, "Procedure for Feeding Animals"). Water bottles (one water bottle/cage) will be provided with tap water throughout the study

Animals will be housed in polycarbonate Tecniplast caging with wire top (Tecniplast, Phoenixville, PA) throughout the acclimation/quarantine and in-life period. Animals will be singly housed to prevent damage to the catheters. The rats will be housed in environmentally controlled rooms, with at least 10 air changes per hour. The rooms will be maintained at a temperature of 68.0 °F to 79.0 °F and a relative humidity of 50% ± 20% with a 12-h light/dark cycle per day. Room temperature, humidity, and light cycles (12 hour: 12 hour reversed light / dark) will be monitored by the AmegaView monitoring system 24 h/day or by a Hygrothermograph during the study (see, e.g. SOP MRI-1170, "Temperature and Humidity Monitoring in Animal Rooms").

### Doses

**HI-6, soman, atropine sulfate, atropine methyl nitrate, 2-PAM Cl, and midazolam:** Single doses of HI-6 (IP, 125 mg/kg); soman (SC, 154 µg/kg, 1.4 x LD₅₀), atropine methyl nitrate (IM, 2 mg/kg); atropine sulfate (0.45 mg/kg), 2-PAM Cl (25 mg/kg), and midazolam (IM, 1.8 mg/kg) are selected based on previous experience with the model. The inclusion of HI-6, atropine methyl nitrate, and midazolam are necessary to ensure adequate survival. This regimen is expected to result in convulsions that achieve a Racine score of at least 3 and an acceptable number of survivors for follow-up study.

**RYANODEX^{®}:** In a single-dose tolerability evaluation in rats, IV bolus doses of 12, 40, and 60 mg/kg RYANODEX^{®} will be given to separate groups of jugular vein cannulated rats. RYANODEX^{®} doses of 10 and 30 mg/kg in rat are equivalent to human doses of approximately 2 and 6 mg/kg, respectively.

### Dosing Scheme

A total of 120 plus up to 20 extra male Sprague-Dawley rats with jugular catheters implanted by the vendor will be used in 6 groups as shown in the below Table. Animals will be run in squads of 20. All group assignments will be randomly assigned with the stipulation that each dose group be represented by at least one animal in each squad.

**Table. Treatment Assignment by Group**

| Group | Pretreatment HI-6 (IP, 30 min prior to GD challenge) | Soman "GD" (SQ) | Atropine Methyl Nitrate (IM, 1 min post GD) | Midazolam (IM, ~20 minutes post-onset seizure activity) | RYANODEX^{®} IV at 20 or 60 min post-onset of seizures |
|---|---|---|---|---|---|
| A (n=20) | 125 mg/kg | 154 µg/kg (1.4 x LD₅₀) | 2.0 mg/kg | 2.0 mg/kg | 60 min with 10 mg/kg |
| B (n=20) | 125 mg/kg | 154 µg/kg (1.4 x LD₅₀) | 2.0 mg/kg | 2.0 mg/kg | 60 min with 30 mg/kg |
| C (n=20) | 125 mg/kg | 154 µg/kg (1.4 x LD₅₀) | 2.0 mg/kg | 2.0 mg/kg | 20 min with 10 mg/kg |
| D (n=20) | 125 mg/kg | 154 µg/kg (1.4 x LD₅₀) | 2.0 mg/kg | 2.0 mg/kg | 20 min with 30 mg/kg |
| E (n=20) | 125 mg/kg | 154 µg/kg (1.4 x LD₅₀) | 2.0 mg/kg | 2.0 mg/kg | Sterile water (control) or sterile water + mannitol (controle) at 50 min |
| F (N=20) | 0 mg/kg | 0 µg/kg | 0 mg/kg | 0 mg/kg | NA |

| | | | | | |
|---|---|---|---|---|---|
| Note: for groups C and D, RYANUDEX^{®} will be given night after Midazolam injection. | | | | | |

### Dose Preparation

GD will be prepared in ice cold 0.9% Sodium Chloride according to, e.g., SOP MRI-5821 "Preparation of Standards and Samples from Research Development and Testing Evaluation (RDTE) Dilute Solutions".

RYANODEX^{®} will be prepared by adding 5 mL of sterile water for injection (without bacteriostatic agent) to one vial of the lyophilized product.

HI-6 will be prepared to a concentration of 250 mg/mL by adding sterile 0.9% saline in a syringable vial.

Atropine methyl nitrate will be prepared to a concentration of 4 mg/mL by adding sterile Water for Injection (WFI) in a syringable vial.

Atropine sulfate will be prepared to a concentration of .8 mg/mL with sterile saline.

2-PAM Cl will be prepared to a concentration of 100 mg/mL with sterile saline.

Atropine sulfate and 2-PAM Cl will then be admixed 1:1 and administered IM at 0.5 mL/kg.

Midazolam will be procured from a vendor as a 10 mg/mL solution and will be administered, as is.

### Experimental Design

### Animal Preparation

Prior to or on Study Day 1 (day of GD exposure), rats will be transferred to the testing location.

### Dosing

On Study Day 1, all animals will be weighted to the nearest gram and all treatment doses will be calculated based on those weights. Groups A - E will receive the cholinesterase reactivator, HI-6 (125 mg/kg) via the intraperitoneal (IP) route 30 minutes prior to GD challenge. All rats in Groups A-E will be exposed to GD (154 µg/kg (308 ug/mL) ~1.4 x LD₅₀) by a single subcutaneous injection. Approximately one minute post-GD injection for Groups A-E, atropine methyl nitrate (AMN) will be administered via IM route (2.0 mg/kg). Approximately 20 minutes post-onset of seizure activity following GD exposure as defined in the Table above, all animals in Groups A-E will receive atropine sulfate (0.45 mg/kg) admixed with 2-PAM-Cl (25 mg/kg) intramuscularly and will receive midazolam at a dose of 1.8 mg/kg intramuscularly. Group F will receive no HI-6, no GD, no AMN, no Midazolam, and no RYANODEX^{®}. RYANODEX^{®} will be administered via a 100 uL Hamilton microsyringe or Luer lock syrine (1 cc), depending on the dose volume that will be administered, then the catheter flushed with at least 0.2 mL of sterile water for injection to ensure complete delivery to the vein.

Initiation of seizure activity was defined by a score of "3" on the Racine scale as described below:
1= immobilization & staring
2= head-nodding, "wet dog shakes"
3= forelimb clonus, rhymithic flicking of the ears accompanied by eye blinks and facial clonus
4= bilateral forelimb clonus
5= bilateral forelimb clonus, rearing and loss of balance

If a rat does not achieve this score of "3" after approximately 20 minutes following GD exposure, it will be replaced in the study.

As shown in the Table above, rats in Groups A and C will receive a 10 mg/kg dose of RYANODEX^{®} IV and rats in Groups B and D received a 30 mg/kg dose of RYANODEX^{®} IV either at 60 or 20 min post-onset of seizures (Racine scale of "3"). Group E rats will receive a dose of control (sterile water or 5% mannitol solution (the amount of mannitol delivered by a 30 mg/kg dose of RYANODEX) in sterile water for injection) IV at a volume comparable to the highest dose of RYANODEX^{®} at 60 min post-onset of seizures.

At approximately 6 hours post-GD challenge for Groups A-E, rats will be administered 5 mL of Lactate Ringers Solution to aid recovery.

### Body Weights

Body weights will be recorded for each animal on study within the first 72 h of receipt from the vendor, then again on Study Days 1 and 2. Any animal that does not gain weight between the first weighting at receipt and the weighing on Study Day 1 will be replaced

### Clinical Observations

General health observations will be performed daily through the quarantine and acclimation period. Animals will be handled twice daily throughout the quarantine and acclimation period and through the entire in life. On Study Day 1, the rats will be observed and wighedprior to exposure. After GD exposure, the rats will be monitored continuously to document the onset of seizures. After midazolam, RYANODEX^{®} and/or control solution dosing, the animals will be observed at 1, 2, 4, and 6 hours after seizure onset, and at least once on the day of euthanasia. Clinical observations including hunched posture, dehydration, rough coat and inappetence will be evaluated and recorded. The Racine score will be recorded at each observation point, if applicable. Any other abnormal clinical observations will also be recorded at this time in the study notebook or appropriate data capture form as per, e.g., SOP MRI-1528 "Procedure for Observation of Animals". Time of death was documented; any animals found dead will be documented at the time they are found as such.

### Euthanasia and Tissue Harvest and Processing

For each group on Study Day 2, all surviving rats will be deeply anesthetized via an IP injection os >75 mg/kg pentobarbital solution. Rats will be perfused transcardially with heparinized saline followed by 0.4% paraformaldehyde, or 10% neutral buffered formalin (perfusion protocol follows Rao, et al (2006). The heart of each rat will be collected and stored in fixative for possible evaluation.

Following tissue collection, animal carcasses will be disposed of according to e.g., SOP MRI-1526 "Disposal of Animal Carcasses and Medical Waste in the Spencer Animal Care Facility".

The brain will be stored overnight at 4°C paragormaldehyde, the next day, it will be drained and replaced with cold PBS and kept in 4 C overnight. The following day, the PBS will be drained and replaced with 10% sucrose solution and stored at 4 C overnight, and then transferred to 10% neutral buffered formalin overnight. Alternatively, the brain will remain in 10% formalin fixative for at least 4 days at room temperature and then shipped. Each brain will be uniformly trimmed into 7 sections as described in Rao, et al (2014) and Bolon, et al (2013) to obtain maximum anatomic presentation of the entire brain, including areas known to be affected by soman and other organophosphorus nerve agents (eg, piriform cortex, entorhinal cortex, hippocampus, amygdala, thalamus, cerebellum). The sections will be routinely processed, sectioned at 5 microns, embedded in paraffin, and stained with hematoxylin and eosin. Personnel will be blinded to dose groups and treatment.

### Neuropathology

The 7 brain sections from each rat will be evaluated microscopically using a 6-point semi-quantitative scoring system. Microscopic lesions will be graded on a 6-point scale.

### Neurobehavioral Tests

Optionally, prior to euthanasia, but after Day 1 GD exposure, rats will be subject the neurobehanioral tests such as those described in Example 1.

## Claims

1. A pharmaceutical composition comprising a therapeutically effective amount of dantrolene or a pharmaceutically acceptable salt thereof, for use in a method of protecting a human subject from neural necrosis after the human subject has been exposed to an organophosphate nerve agent, wherein the fronto-parietal cortex is protected from neural necrosis;
wherein the therapeutically effective amount is 1 mg/kg to 30 mg/kg dantrolene;
wherein the method comprises administering to the human subject said pharmaceutical composition;
wherein at least one dose of the dantrolene or pharmaceutically acceptable salt thereof is administered to the human subject 24 hours or less after the subject has been exposed to the nerve agent; and
wherein the pharmaceutical composition comprises dantrolene or a pharmaceutically acceptable salt thereof, mannitol, a polysorbate, a povidone, an optional pH adjustor, and water.

2. A pharmaceutical composition comprising a therapeutically effective amount of dantrolene or a pharmaceutically acceptable salt thereof, for use in a method of treating a human subject exposed to an organophosphate nerve agent, wherein the fronto-parietal cortex is protected from neural necrosis;
wherein the therapeutically effective amount is 1 mg/kg to 30 mg/kg dantrolene;
wherein the method comprises administering said pharmaceutical composition to the human subject;
wherein at least one dose of the dantrolene or pharmaceutically acceptable salt thereof is administered to the human subject 24 hours or less after the subject has been exposed to the nerve agent; and
wherein the pharmaceutical composition comprises dantrolene or a pharmaceutically acceptable salt thereof, mannitol, a polysorbate, a povidone, an optional pH adjustor, and water.

3. A pharmaceutical composition comprising a therapeutically effective amount of dantrolene or a pharmaceutically acceptable salt thereof, for use in a method of protecting a human subject from a decrease in central nervous system function resulting from exposure to an organophosphate nerve agent, wherein the fronto-parietal cortex is protected from neural necrosis;
wherein the therapeutically effective amount is 1 mg/kg to 30 mg/kg dantrolene;
wherein the method comprises administering said pharmaceutical composition to the human subject;
wherein at least one dose of the dantrolene or pharmaceutically acceptable salt thereof is administered to the human subject 24 hours or less after the subject has been exposed to the nerve agent; and
wherein the pharmaceutical composition comprises dantrolene or a pharmaceutically acceptable salt thereof, mannitol, a polysorbate, a povidone, an optional pH adjustor, and water.

4. A pharmaceutical composition comprising a therapeutically effective amount of dantrolene or a pharmaceutically acceptable salt thereof, for use in a method of treating organophosphate nerve agent-induced seizures, in particular status epilepticus, wherein the fronto-parietal cortex is protected from neural necrosis;
wherein the method comprises administering said pharmaceutical composition to the human subject;
wherein the therapeutically effective amount is 1 mg/kg to 30 mg/kg dantrolene;
wherein at least one dose of the dantrolene or pharmaceutically acceptable salt thereof is administered to the human subject 24 hours or less after the subject has been exposed to the nerve agent; and
wherein the pharmaceutical composition comprises dantrolene or a pharmaceutically acceptable salt thereof, mannitol, a polysorbate, a povidone, an optional pH adjustor, and water.

5. A pharmaceutical composition comprising a therapeutically effective amount of dantrolene or a pharmaceutically acceptable salt thereof, for use in a method of protecting a human subject from a central nervous system dysfunction resulting from exposure to an organophosphate nerve agent, wherein the fronto-parietal cortex is protected from neural necrosis;
wherein the method comprises administering said pharmaceutical composition to the human subject;
wherein the therapeutically effective amount is 1 mg/kg to 30 mg/kg dantrolene;
wherein at least one dose of the dantrolene or pharmaceutically acceptable salt thereof is administered to the human subject 24 hours or less after the subject has been exposed to the nerve agent; and
wherein the pharmaceutical composition comprises dantrolene or a pharmaceutically acceptable salt thereof, mannitol, a polysorbate, a povidone, an optional pH adjustor, and water.

6. A pharmaceutical composition comprising a therapeutically effective amount of dantrolene or a pharmaceutically acceptable salt thereof, for use in a method of treating behavioral changes resulting from exposure to an organophosphate nerve agent, wherein the fronto-parietal cortex is protected from neural necrosis;
wherein the method comprises administering said pharmaceutical composition to the human subject;
wherein the therapeutically effective amount is 1 mg/kg to 30 mg/kg dantrolene;
wherein at least one dose of the dantrolene or pharmaceutically acceptable salt thereof is administered of to the human subject 24 hours or less after the subject has been exposed to the nerve agent; and
wherein the pharmaceutical composition comprises dantrolene or a pharmaceutically acceptable salt thereof, mannitol, a polysorbate, a povidone, an optional pH adjustor, and water.

7. The pharmaceutical composition for use of any one of the preceding claims, wherein:
(i) the nerve agent is O-pinacolyl methylphosphonofluoridate (soman), ethyl N,N-dimethylphosphoramidocyanidate (tabun), propan-2-yl methylphosphonofluoridate (sarin), cyclohexyl methylphosphonofluoridate (cyclosarin), or 2-(Dimethylamino)ethyl (GV); or
(ii) the nerve agent is O-cyclopentyl S-(2-diethylaminoethyl) methylphosphonothiolate (EA-3148), (S)-(ethyl {[2-(diethylamino)ethyl]sulfanyl}(ethyl)phosphinate) (VE), O,O-Diethyl S-[2-(diethylamino)ethyl] phosphorothioate (VG), S-[2-(Diethylamino)ethyl] O-ethyl methylphosphonothioate (VM), N,N-diethyl-2-(methyl-(2-methylpropoxy)phosphoryl)sulfanylethanamine (VR), or Ethyl ({2-[bis(propan-2-yl)amino]ethyl}sulfanyl)(methyl)phosphinate (VX).

8. The pharmaceutical composition for use of any one of the preceding claims, further comprising administering to the human subject an acetylcholinesterase reactivator, a reverse antagonist of acetylcholine receptors, an anti-seizure medication, or a combination thereof, wherein:
(i) the acetylcholinesterase reactivator is asoxime chloride (HI-6);
(ii) the reverse antagonist of acetylcholine receptors is atropine methyl nitrate, optionally wherein the benzodiazepine is midazolam; and
(iii) the anti-seizure medication is a benzodiazepine;
optionally wherein pharmaceutical composition is administered after the administration of the acetylcholinesterase reactivator.

9. The pharmaceutical composition for use of claim 8, wherein the pharmaceutical composition is administered after the administration of the acetylcholinesterase reactivator and after the administration of the reverse antagonist of acetylcholine receptors, optionally wherein:
(i) the pharmaceutical composition is administered concurrently or substantially concurrently with the administration of the anti-seizure medication; or
(ii) the pharmaceutical composition is administered after the administration of the anti-seizure medication.

10. The pharmaceutical composition for use of any one of the preceding claims, wherein the pharmaceutical composition is administered intravenously, subcutaneously, intramuscularly, intraosseously, or transdermally.

11. The pharmaceutical composition for use of any one of the preceding claims, wherein the administration of the pharmaceutical composition comprising dantrolene or a pharmaceutically acceptable salt thereof results in improved neurobehavioral performance, as compared to a subject exposed to the nerve agent that was not administered the pharmaceutical composition comprising dantrolene or a pharmaceutically acceptable salt thereof.

## Patentansprüche

1. Pharmazeutische Zusammensetzung, umfassend eine therapeutisch wirksame Menge an Dantrolen oder einem pharmazeutisch annehmbaren Salz davon, zur Verwendung in einem Verfahren zum Schützen eines menschlichen Individuums vor neuraler Nekrose, nachdem das menschliche Individuum einem Organophosphat-Nervenkampfstoff ausgesetzt war, wobei der frontoparietale Kortex vor neuraler Nekrose geschützt wird;
wobei die therapeutisch wirksame Menge 1 mg/kg bis 30 mg/kg Dantrolen beträgt;
wobei das Verfahren das Verabreichen der pharmazeutischen Zusammensetzung an das menschliche Individuum umfasst; wobei mindestens eine Dosis des Dantrolen oder des pharmazeutisch annehmbaren Salzes davon 24 Stunden oder weniger, nachdem das Individuum dem Nervenkampfstoff ausgesetzt war, an das menschliche Individuum verabreicht wird; und
wobei die pharmazeutische Zusammensetzung Dantrolen oder ein pharmazeutisch annehmbares Salz davon, Mannit, ein Polysorbat, ein Povidon, ein optionales Mittel zum Einstellen des pH-Werts und Wasser umfasst.

2. Pharmazeutische Zusammensetzung, umfassend eine therapeutisch wirksame Menge an Dantrolen oder einem pharmazeutisch annehmbaren Salz davon, zur Verwendung in einem Verfahren zum Behandeln eines menschlichen Individuums, das einem Organophosphat-Nervenkampfstoff ausgesetzt war, wobei der frontoparietale Kortex vor neuraler Nekrose geschützt wird;
wobei die therapeutisch wirksame Menge 1 mg/kg bis 30 mg/kg Dantrolen beträgt;
wobei das Verfahren das Verabreichen der pharmazeutischen Zusammensetzung an das menschliche Individuum umfasst; wobei mindestens eine Dosis des Dantrolen oder des pharmazeutisch annehmbaren Salzes davon 24 Stunden oder weniger, nachdem das Individuum dem Nervenkampfstoff ausgesetzt war, an das menschliche Individuum verabreicht wird; und
wobei die pharmazeutische Zusammensetzung Dantrolen oder ein pharmazeutisch annehmbares Salz davon, Mannit, ein Polysorbat, ein Povidon, ein optionales Mittel zum Einstellen des pH-Werts und Wasser umfasst.

3. Pharmazeutische Zusammensetzung, umfassend eine therapeutisch wirksame Menge an Dantrolen oder einem pharmazeutisch annehmbaren Salz davon, zur Verwendung in einem Verfahren zum Schützen eines menschlichen Individuums vor einer Minderung der Funktion des Zentralnervensystems infolge einer Exposition gegenüber einem Organophosphat-Nervenkampfstoff, wobei der frontoparietale Kortex vor neuraler Nekrose geschützt wird;
wobei die therapeutisch wirksame Menge 1 mg/kg bis 30 mg/kg Dantrolen beträgt;
wobei das Verfahren das Verabreichen der pharmazeutischen Zusammensetzung an das menschliche Individuum umfasst; wobei mindestens eine Dosis des Dantrolen oder des pharmazeutisch annehmbaren Salzes davon 24 Stunden oder weniger, nachdem das Individuum dem Nervenkampfstoff ausgesetzt war, an das menschliche Individuum verabreicht wird; und
wobei die pharmazeutische Zusammensetzung Dantrolen oder ein pharmazeutisch annehmbares Salz davon, Mannit, ein Polysorbat, ein Povidon, ein optionales Mittel zum Einstellen des pH-Werts und Wasser umfasst.

4. Pharmazeutische Zusammensetzung, umfassend eine therapeutisch wirksame Menge an Dantrolen oder einem pharmazeutisch annehmbaren Salz davon, zur Verwendung in einem Verfahren zum Behandeln durch Organophosphat-Nervenkampfstoff ausgelöster Anfälle, insbesondere Status epilepticus, wobei der frontoparietale Kortex vor neuraler Nekrose geschützt wird;
wobei das Verfahren das Verabreichen der pharmazeutischen Zusammensetzung an das menschliche Individuum umfasst; wobei die therapeutisch wirksame Menge 1 mg/kg bis 30 mg/kg Dantrolen beträgt;
wobei mindestens eine Dosis des Dantrolen oder des pharmazeutisch annehmbaren Salzes davon 24 Stunden oder weniger, nachdem das Individuum dem Nervenkampfstoff ausgesetzt war, an das menschliche Individuum verabreicht wird; und
wobei die pharmazeutische Zusammensetzung Dantrolen oder ein pharmazeutisch annehmbares Salz davon, Mannit, ein Polysorbat, ein Povidon, ein optionales Mittel zum Einstellen des pH-Werts und Wasser umfasst.

5. Pharmazeutische Zusammensetzung, umfassend eine therapeutisch wirksame Menge an Dantrolen oder einem pharmazeutisch annehmbaren Salz davon, zur Verwendung in einem Verfahren zum Schützen eines menschlichen Individuums vor einer Dysfunktion des Zentralnervensystems infolge einer Exposition gegenüber einem Organophosphat-Nervenkampfstoff, wobei der frontoparietale Kortex vor neuraler Nekrose geschützt wird;
wobei das Verfahren das Verabreichen der pharmazeutischen Zusammensetzung an das menschliche Individuum umfasst; wobei die therapeutisch wirksame Menge 1 mg/kg bis 30 mg/kg Dantrolen beträgt;
wobei mindestens eine Dosis des Dantrolen oder des pharmazeutisch annehmbaren Salzes davon 24 Stunden oder weniger, nachdem das Individuum dem Nervenkampfstoff ausgesetzt war, an das menschliche Individuum verabreicht wird; und
wobei die pharmazeutische Zusammensetzung Dantrolen oder ein pharmazeutisch annehmbares Salz davon, Mannit, ein Polysorbat, ein Povidon, ein optionales Mittel zum Einstellen des pH-Werts und Wasser umfasst.

6. Pharmazeutische Zusammensetzung, umfassend eine therapeutisch wirksame Menge an Dantrolen oder einem pharmazeutisch annehmbaren Salz davon, zur Verwendung in einem Verfahren zum Behandeln von aus einer Exposition gegenüber einem Organophosphat-Nervenkampfstoff resultierenden Verhaltensänderungen, wobei der frontoparietale Kortex vor neuraler Nekrose geschützt wird;
wobei das Verfahren das Verabreichen der pharmazeutischen Zusammensetzung an das menschliche Individuum umfasst; wobei die therapeutisch wirksame Menge 1 mg/kg bis 30 mg/kg Dantrolen beträgt;
wobei mindestens eine Dosis des Dantrolen oder des pharmazeutisch annehmbaren Salzes davon 24 Stunden oder weniger, nachdem das Individuum dem Nervenkampfstoff ausgesetzt war, an das menschliche Individuum verabreicht wird; und
wobei die pharmazeutische Zusammensetzung Dantrolen oder ein pharmazeutisch annehmbares Salz davon, Mannit, ein Polysorbat, ein Povidon, ein optionales Mittel zum Einstellen des pH-Werts und Wasser umfasst.

7. Pharmazeutische Zusammensetzung zur Verwendung nach einem der vorstehenden Ansprüche, wobei:
(i) der Nervenkampfstoff O-Pinacolylmethylphosphonofluoridat (Soman), Ethyl-N,N-dimethylphosphoramidocyanidat (Tabun), Propan-2-ylmethylphosphonofluoridat (Sarin), Cyclohexylmethylphosphonofluoridat (Cyclosarin) oder 2-(Dimethylamino)ethyl (GV) ist; oder
(ii) der Nervenkampfstoff O-Cyclopentyl-S-(2-diethylaminoethyl)methylphosphonothiolat (EA-3148), (S)-(Ethyl-{[2-(diethylamino)ethyl]sulfanyl}(ethyl)phosphinat) (VE), O,O-Diethyl-S-[2-(diethylamino)ethyl]phosphorothioat (VG), S-[2-(Diethylamino)ethyl]O-ethylmethylphosphonothioat (VM), N,N-Diethyl-2-(methyl-(2-methylpropoxy)phosphoryl)sulfanylethanamin (VR) oder Ethyl-({2-[bis(propan-2-yl)amino]ethyl}sulfanyl)(methyl)phosphinat (VX) ist.

8. Pharmazeutische Zusammensetzung zur Verwendung nach einem der vorstehenden Ansprüche, ferner umfassend das Verabreichen eines Acetylcholinesterase-Reaktivators, eines reversen Antagonisten von Acetylcholinrezeptoren, eines Anfallssuppressivums oder einer Kombination davon an ein menschliches Individuum, wobei:
(i) der Acetylcholinesterase-Reaktivator Asoximchlorid (HI-6) ist;
(ii) der reverse Antagonist von Acetylcholinrezeptoren Atropinmethylnitrat ist, wobei gegebenenfalls das Benzodiazepin Midazolam ist; und
(iii)das Anfallssuppressivum ein Benzodiazepin ist;
wobei gegebenenfalls die pharmazeutische Zusammensetzung nach der Verabreichung des Acetylcholinesterase-Reaktivators verabreicht wird.

9. Pharmazeutische Zusammensetzung zur Verwendung nach Anspruch 8, wobei die pharmazeutische Zusammensetzung nach der Verabreichung des Acetylcholinesterase-Reaktivators und nach der Verabreichung des reversen Antagonisten von Acetylcholinrezeptoren verabreicht wird, wobei gegebenenfalls:
(i) die pharmazeutische Zusammensetzung gleichzeitig oder im Wesentlichen gleichzeitig mit der Verabreichung des Anfallssuppressivums verabreicht wird; oder
(ii) die pharmazeutische Zusammensetzung nach der Verabreichung des Anfallssuppressivums verabreicht wird.

10. Pharmazeutische Zusammensetzung zur Verwendung nach einem der vorstehenden Ansprüche, wobei die pharmazeutische Zusammensetzung intravenös, subkutan, intramuskulär, intraossär oder transdermal verabreicht wird.

11. Pharmazeutische Zusammensetzung zur Verwendung nach einem der vorstehenden Ansprüche, wobei die Verabreichung der pharmazeutischen Zusammensetzung, die Dantrolen oder ein pharmazeutisch annehmbares Salz davon umfasst, im Vergleich zu einem dem Nervenkampfstoff ausgesetzten Individuum, an das die pharmazeutische Zusammensetzung, die Dantrolen oder ein pharmazeutisch annehmbares Salz davon umfasst, nicht verabreicht wurde, eine verbesserte neurobehaviorale Leistung zur Folge hat.

## Revendications

1. Composition pharmaceutique comprenant une quantité thérapeutiquement efficace de dantrolène ou d'un sel pharmaceutiquement acceptable de celui-ci, pour une utilisation dans un procédé de protection d'un sujet humain contre la nécrose neuronale après que le sujet humain a été exposé à un agent neurotoxique de type organophosphate, dans laquelle le cortex fronto-pariétal est protégé contre la nécrose neuronale ;
dans laquelle la quantité thérapeutiquement efficace est de 1 mg/kg à 30 mg/kg de dantrolène ;
dans laquelle le procédé comprend l'administration au sujet humain de ladite composition pharmaceutique ; dans laquelle au moins une dose du dantrolène ou d'un sel pharmaceutiquement acceptable de celui-ci est administrée au sujet humain 24 heures ou moins après que le sujet a été exposé à l'agent neurotoxique ; et
dans laquelle la composition pharmaceutique comprend du dantrolène ou un sel pharmaceutiquement acceptable de celui-ci, du mannitol, un polysorbate, une povidone, un ajusteur de pH éventuel et de l'eau.

2. Composition pharmaceutique comprenant une quantité thérapeutiquement efficace de dantrolène ou d'un sel pharmaceutiquement acceptable de celui-ci, pour une utilisation dans un procédé de traitement d'un sujet humain exposé à un agent neurotoxique de type organophosphate, dans laquelle le cortex fronto-pariétal est protégé contre la nécrose neuronale ;
dans laquelle la quantité thérapeutiquement efficace est de 1 mg/kg à 30 mg/kg de dantrolène ;
dans laquelle le procédé comprend l'administration de ladite composition pharmaceutique au sujet humain ; dans laquelle au moins une dose du dantrolène ou d'un sel pharmaceutiquement acceptable de celui-ci est administrée au sujet humain 24 heures ou moins après que le sujet a été exposé à l'agent neurotoxique ; et
dans laquelle la composition pharmaceutique comprend du dantrolène ou un sel pharmaceutiquement acceptable de celui-ci, du mannitol, un polysorbate, une povidone, un ajusteur de pH éventuel et de l'eau.

3. Composition pharmaceutique comprenant une quantité thérapeutiquement efficace de dantrolène ou d'un sel pharmaceutiquement acceptable de celui-ci, pour une utilisation dans un procédé de protection d'un sujet humain contre une diminution de la fonction du système nerveux central résultant d'une exposition à un agent neurotoxique de type organophosphate, dans laquelle le cortex fronto-pariétal est protégé contre la nécrose neuronale ;
dans laquelle la quantité thérapeutiquement efficace est de 1 mg/kg à 30 mg/kg de dantrolène ;
dans laquelle le procédé comprend l'administration de ladite composition pharmaceutique au sujet humain ; dans laquelle au moins une dose du dantrolène ou d'un sel pharmaceutiquement acceptable de celui-ci est administrée au sujet humain 24 heures ou moins après que le sujet a été exposé à l'agent neurotoxique ; et
dans laquelle la composition pharmaceutique comprend du dantrolène ou un sel pharmaceutiquement acceptable de celui-ci, du mannitol, un polysorbate, une povidone, un ajusteur de pH éventuel et de l'eau.

4. Composition pharmaceutique comprenant une quantité thérapeutiquement efficace de dantrolène ou d'un sel pharmaceutiquement acceptable de celui-ci, pour une utilisation dans un procédé de traitement de crises induites par un agent neurotoxique de type organophosphate, en particulier un état épileptique, dans laquelle le cortex fronto-pariétal est protégé contre la nécrose neuronale ;
dans laquelle le procédé comprend l'administration de ladite composition pharmaceutique au sujet humain ; dans laquelle la quantité thérapeutiquement efficace est de 1 mg/kg à 30 mg/kg de dantrolène ;
dans laquelle au moins une dose du dantrolène ou d'un sel pharmaceutiquement acceptable de celui-ci est administrée au sujet humain 24 heures ou moins après que le sujet a été exposé à l'agent neurotoxique ; et
dans laquelle la composition pharmaceutique comprend du dantrolène ou un sel pharmaceutiquement acceptable de celui-ci, du mannitol, un polysorbate, une povidone, un ajusteur de pH éventuel et de l'eau.

5. Composition pharmaceutique comprenant une quantité thérapeutiquement efficace de dantrolène ou d'un sel pharmaceutiquement acceptable de celui-ci, pour une utilisation dans un procédé de protection d'un sujet humain contre un dysfonctionnement du système nerveux central résultant d'une exposition à un agent neurotoxique de type organophosphate, dans laquelle le cortex fronto-pariétal est protégé contre la nécrose neuronale ;
dans laquelle le procédé comprend l'administration de ladite composition pharmaceutique au sujet humain ; dans laquelle la quantité thérapeutiquement efficace est de 1 mg/kg à 30 mg/kg de dantrolène ;
dans laquelle au moins une dose du dantrolène ou d'un sel pharmaceutiquement acceptable de celui-ci est administrée au sujet humain 24 heures ou moins après que le sujet a été exposé à l'agent neurotoxique ; et
dans laquelle la composition pharmaceutique comprend du dantrolène ou un sel pharmaceutiquement acceptable de celui-ci, du mannitol, un polysorbate, une povidone, un ajusteur de pH éventuel et de l'eau.

6. Composition pharmaceutique comprenant une quantité thérapeutiquement efficace de dantrolène ou d'un sel pharmaceutiquement acceptable de celui-ci, pour une utilisation dans un procédé de traitement de changements comportementaux résultant d'une exposition à un agent neurotoxique de type organophosphate, dans laquelle le cortex fronto-pariétal est protégé contre la nécrose neuronale;
dans laquelle le procédé comprend l'administration de ladite composition pharmaceutique au sujet humain ; dans laquelle la quantité thérapeutiquement efficace est de 1 mg/kg à 30 mg/kg de dantrolène ;
dans laquelle au moins une dose du dantrolène ou du sel pharmaceutiquement acceptable de celui-ci est administrée au sujet humain 24 heures ou moins après que le sujet a été exposé à l'agent neurotoxique ; et
dans laquelle la composition pharmaceutique comprend du dantrolène ou un sel pharmaceutiquement acceptable de celui-ci, du mannitol, un polysorbate, une povidone, un ajusteur de pH éventuel et de l'eau.

7. Composition pharmaceutique pour une utilisation selon l'une quelconque des revendications précédentes, dans laquelle :
(i) l'agent neurotoxique est le méthylphosphonofluoridate de O-pinacolyle (soman), le N,N-diméthylphosphoramidocyanidate d'éthyle (tabun), le méthylphosphonofluoridate de propan-2-yl (sarin), le méthylphosphonofluoridate de cyclohexyle (cyclosarin) ou le le 2-(diméthylamino)éthyle (GV) ; ou
(ii) l'agent neurotoxique est le méthylphosphonothiolate de O-cyclopentyle S-(2-diéthylaminoéthyle) (EA-3148), le (S)-({[2-(diéthylamino)éthyl]sulfanyl}(éthyl)phosphinate d'éthyle) (VE), le phosphorothioate de O,O-diéthyle S-[2-(diéthylamino)éthyle] (VG), le méthylphosphonothioate de S-[2-(diéthylamino)éthyle] O-éthyle (VM), le N,N-diéthyl-2-(méthyl-(2-méthylpropoxy)phosphoryl)sulfanylethanamine (VR) ou le ({2-[bis(propan-2-yl)amino]éthyl}sulfanyl) (méthyl)phosphinate d'éthyle (VX).

8. Composition pharmaceutique pour une utilisation selon l'une quelconque des revendications précédentes, comprenant en outre l'administration au sujet humain d'un réactivateur d'acétylcholinestérase, d'un antagoniste inverse de récepteurs d'acétylcholine, d'un médicament antiépileptique, ou d'une combinaison de ceux-ci, dans laquelle :
(i) le réactivateur d'acétylcholinestérase est le chlorure d'asoxime (HI-6) ;
(ii) l'antagoniste inverse de récepteurs d'acétylcholine est le nitrate de méthylatropine, éventuellement dans laquelle la benzodiazépine est le midazolam ; et
(iii) le médicament antiépileptique est une benzodiazépine ;
éventuellement dans laquelle la composition pharmaceutique est administrée après l'administration du réactivateur d'acétylcholinestérase.

9. Composition pharmaceutique pour une utilisation selon la revendication 8, dans laquelle la composition pharmaceutique est administrée après l'administration du réactivateur d'acétylcholinestérase et après l'administration de l'antagoniste inverse de récepteurs d'acétylcholine, éventuellement dans laquelle :
(i) la composition pharmaceutique est administrée simultanément ou sensiblement simultanément au médicament antiépileptique ; ou
(ii) la composition pharmaceutique est administrée après l'administration du médicament antiépileptique.

10. Composition pharmaceutique pour une utilisation selon l'une quelconque des revendications précédentes, dans laquelle la composition pharmaceutique est administrée par voie intraveineuse, sous-cutanée, intramusculaire, intraosseuse, ou transdermique.

11. Composition pharmaceutique pour une utilisation selon l'une quelconque des revendications précédentes, dans laquelle l'administration de la composition pharmaceutique comprenant du dantrolène ou un sel pharmaceutiquement acceptable de celui-ci conduit à une performance neuro-comportementale améliorée, par comparaison avec un sujet exposé à l'agent neurotoxique qui n'a pas reçu la composition pharmaceutique comprenant du dantrolène ou un sel pharmaceutiquement acceptable de celui-ci.
